# EUROPEAN PATENT APPLICATION

(11) **EP 4 317 187 A1**
(43) Date of publication of application: **07.02.2024**
(21) Application number: 22779039.1
(22) Date of filing: 30.03.2022
(51) Int. Cl.: C07K 16/28, C12N 15/63, C12N 15/13, A61K 39/395, A61P 13/12

(54) **ANTIBODY CAPABLE OF SPECIFICALLY BINDING TO HUMAN ENDOTHELIN RECEPTOR, AND USE THEREOF IN TREATMENT OF DIABETIC NEPHROPATHY AND CHRONIC NEPHROPATHY**

(30) Priority: 31.03.2021 CN 202110353110
(71) Applicant: Gmax Biopharm LLC, Hangzhou, Zhejiang 310052 (CN)
(72) Inventor: ZHANG, Cheng, Hangzhou, Zhejiang 310052 (CN); JING, Shuqian, Hangzhou, Zhejiang 310052 (CN)
(74) Representative: Simmons & Simmons LLP (Munich)
(86) International application number: PCT/CN2022/084164
(87) International publication number: WO 2022/206857

(57) **Abstract**

Provided herein are an ET_{A}R antibody and a pharmaceutical composition thereof, also provided herein is a method for treating, preventing or ameliorating one or more symptoms of diabetic nephropathy and one or more symptoms of chronic nephropathy.

## Description

### Technical Field

Provided herein are an ET_{A}R antibody and a pharmaceutical composition thereof. Also provided herein is a method for treating, preventing or ameliorating one or more symptoms of diabetic nephropathy and one or more symptoms of chronic nephropathy.

### Background Art

Endothelin is a polypeptide consisting of 21 amino acids with a molecular weight of 2,400 Da, the N-terminus comprises cysteines at positions 1-15 and 3-11 which are linked by two disulfide bonds, and the C-terminus comprises some hydrophobic amino acid residues. The N-terminal structure determines the affinity of endothelin to a receptor, while the C-terminal structure determines the position where endothelin binds to the receptor. There are three subtypes of endothelin (ET), namely ET-1, ET-2, and ET-3. Endothelin receptors (ETRs) include two types: ET_{A}R and ET_{B}R, which belong to G-protein coupled receptors (GPCRs). ET and receptors thereof are widely distributed in human tissues and organs, including cardiovascular system, lungs, renal system, brain, nerve tissues, etc.. Renal medulla contains the highest level of ET-1 in the body (Kitamura et al., 1989, BiochemBiophys Res Commun 161: 348-352). Compared with other organs, kidneys are more sensitive to ET-1 (Speed and Pollock, 2013, Hypertension 61: 1142-1145). ET and receptors thereof may be involved in renal injury caused by obesity, hypertension, diabetes, etc., and generally mediated by ET_{A}R.

With the development of global economy and the improvement of living standards, the incidence and prevalence of diabetes increase progressively, so that diabetes has become a significant public health problem. Diabetic nephropathy is one of the most important complications of diabetes, affecting 15-25% of patients with type I diabetes and 30-40% of patients with type II diabetes (Schernthaner and Schernthaner, 2013, J Nephrol 26:975-985).

### Summary of the Invention

Provided herein are an ET_{A}R antibody and a pharmaceutical composition thereof. Also provided herein is a method for treating, preventing or ameliorating one or more symptoms of diabetic nephropathy and one or more symptoms of chronic nephropathy.

Provided herein are an ET_{A}R antibody, which comprises one, two, three, four, five or six amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30;
b. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48;
c. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68;
d. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90;
e. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114; and
f. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

In one embodiment, the ET_{A}R antibody provided herein comprises:
a. a light chain CDR1 amino acid sequence: SEQ ID NO: 8
b. a light chain CDR2 amino acid sequence: SEQ ID NO: 32
c. a light chain CDR3 amino acid sequence: SEQ ID NO: 50
d. a heavy chain CDR1 amino acid sequence: SEQ ID NO: 70
e. a heavy chain CDR2 amino acid sequence: SEQ ID NO: 92
f. a heavy chain CDR3 amino acid sequence: SEQ ID NO: 116.

In one embodiment, the ET_{A}R antibody provided herein comprises:
a. a light chain CDR1 amino acid sequence: SEQ ID NO: 10
b. a light chain CDR2 amino acid sequence: SEQ ID NO: 34
c. a light chain CDR3 amino acid sequence: SEQ ID NO: 52
d. a heavy chain CDR1 amino acid sequence: SEQ ID NO: 72
e. a heavy chain CDR2 amino acid sequence: SEQ ID NO: 94
f. a heavy chain CDR3 amino acid sequence: SEQ ID NO: 118.

In one embodiment, the ET_{A}R antibody provided herein comprises:
a. a light chain CDR1 amino acid sequence: SEQ ID NO: 12
b. a light chain CDR2 amino acid sequence: SEQ ID NO: 36
c. a light chain CDR3 amino acid sequence: SEQ ID NO: 54
d. a heavy chain CDR1 amino acid sequence: SEQ ID NO: 74
e. a heavy chain CDR2 amino acid sequence: SEQ ID NO: 96
f. a heavy chain CDR3 amino acid sequence: SEQ ID NO: 120.

In one embodiment, the ET_{A}R antibody provided herein comprises:
a. a light chain CDR1 amino acid sequence: SEQ ID NO: 14
b. a light chain CDR2 amino acid sequence: SEQ ID NO: 38
c. a light chain CDR3 amino acid sequence: SEQ ID NO: 56
d. a heavy chain CDR1 amino acid sequence: SEQ ID NO: 76
e. a heavy chain CDR2 amino acid sequence: SEQ ID NO: 98
f. a heavy chain CDR3 amino acid sequence: SEQ ID NO: 122.

In one embodiment, the ET_{A}R antibody provided herein comprises:
a. a light chain CDR1 amino acid sequence: SEQ ID NO: 16
b. a light chain CDR2 amino acid sequence: SEQ ID NO: 40
c. a light chain CDR3 amino acid sequence: SEQ ID NO: 58
d. a heavy chain CDR1 amino acid sequence: SEQ ID NO: 78
e. a heavy chain CDR2 amino acid sequence: SEQ ID NO: 100
f. a heavy chain CDR3 amino acid sequence: SEQ ID NO: 124.

In one embodiment, the ET_{A}R antibody provided herein comprises:
a. a light chain CDR1 amino acid sequence: SEQ ID NO: 18
b. a light chain CDR2 amino acid sequence: SEQ ID NO: 42
c. a light chain CDR3 amino acid sequence: SEQ ID NO: 60
d. a heavy chain CDR1 amino acid sequence: SEQ ID NO: 80
e. a heavy chain CDR2 amino acid sequence: SEQ ID NO: 102
f. a heavy chain CDR3 amino acid sequence: SEQ ID NO: 126.

In one embodiment, the ET_{A}R antibody provided herein comprises:
a. a light chain CDR1 amino acid sequence: SEQ ID NO: 20
b. a light chain CDR2 amino acid sequence: SEQ ID NO: 44
c. a light chain CDR3 amino acid sequence: SEQ ID NO: 62
d. a heavy chain CDR1 amino acid sequence: SEQ ID NO: 82
e. a heavy chain CDR2 amino acid sequence: SEQ ID NO: 104
f. a heavy chain CDR3 amino acid sequence: SEQ ID NO: 128.

In one embodiment, the ET_{A}R antibody provided herein comprises:
a. a light chain CDR1 amino acid sequence: SEQ ID NO: 22
b. a light chain CDR2 amino acid sequence: SEQ ID NO: 44
c. a light chain CDR3 amino acid sequence: SEQ ID NO: 62
d. a heavy chain CDR1 amino acid sequence: SEQ ID NO: 82
e. a heavy chain CDR2 amino acid sequence: SEQ ID NO: 106
f. a heavy chain CDR3 amino acid sequence: SEQ ID NO: 128.

In one embodiment, the ET_{A}R antibody provided herein comprises:
a. a light chain CDR1 amino acid sequence: SEQ ID NO: 24
b. a light chain CDR2 amino acid sequence: SEQ ID NO: 44
c. a light chain CDR3 amino acid sequence: SEQ ID NO: 62
d. a heavy chain CDR1 amino acid sequence: SEQ ID NO: 84
e. a heavy chain CDR2 amino acid sequence: SEQ ID NO: 108
f. a heavy chain CDR3 amino acid sequence: SEQ ID NO: 130.

In one embodiment, the ET_{A}R antibody provided herein comprises:
a. a light chain CDR1 amino acid sequence: SEQ ID NO: 26
b. a light chain CDR2 amino acid sequence: SEQ ID NO: 46
c. a light chain CDR3 amino acid sequence: SEQ ID NO: 64
d. a heavy chain CDR1 amino acid sequence: SEQ ID NO: 86
e. a heavy chain CDR2 amino acid sequence: SEQ ID NO: 110
f. a heavy chain CDR3 amino acid sequence: SEQ ID NO: 132.

In one embodiment, the ET_{A}R antibody provided herein comprises:
a. a light chain CDR1 amino acid sequence: SEQ ID NO: 28
b. a light chain CDR2 amino acid sequence: SEQ ID NO: 46
c. a light chain CDR3 amino acid sequence: SEQ ID NO: 66
d. a heavy chain CDR1 amino acid sequence: SEQ ID NO: 88
e. a heavy chain CDR2 amino acid sequence: SEQ ID NO: 112
f. a heavy chain CDR3 amino acid sequence: SEQ ID NO: 134.

In another embodiment, the ET_{A}R antibody provided herein comprises:
a. a light chain CDR1 amino acid sequence: SEQ ID NO: 30
b. a light chain CDR2 amino acid sequence: SEQ ID NO: 48
c. a light chain CDR3 amino acid sequence: SEQ ID NO: 68
d. a heavy chain CDR1 amino acid sequence: SEQ ID NO: 90
e. a heavy chain CDR2 amino acid sequence: SEQ ID NO: 114
f. a heavy chain CDR3 amino acid sequence: SEQ ID NO: 136.

Also provided herein is a pharmaceutical composition, comprising an ET_{A}R antibody provided herein in combination with one or more pharmaceutically acceptable carriers.

Provided herein is a method for treating, preventing or ameliorating one or more symptoms of diabetic nephropathy, comprising administering to a subject a therapeutically effective amount of the pharmaceutical composition provided herein.

Provided herein is a method for treating, preventing or ameliorating one or more symptoms of chronic nephropathy, comprising administering to a subject a therapeutically effective amount of the pharmaceutical composition provided herein.

Provided herein is a method for treating, preventing or ameliorating one or more symptoms of a disease accompanied by proteinuria, comprising administering to a subject a therapeutically effective amount of the pharmaceutical composition provided herein.

Provided herein is a method for treating, preventing or ameliorating one or more symptoms of a disease accompanied by a decrease in glomerular filtration rate, comprising administering to a subject a therapeutically effective amount of the pharmaceutical composition provided herein.

Provided herein is a kit for treating diabetic nephropathy, chronic nephropathy, and a disease accompanied by proteinuria or a disease accompanied by a decrease in glomerular filtration rate, comprising the pharmaceutical composition provided herein.

Provided herein is the use of the pharmaceutical composition in the preparation of a drug for treating diabetic nephropathy, chronic nephropathy, and a disease accompanied by proteinuria or a disease accompanied by a decrease in glomerular filtration rate. Provided herein is an isolated nucleic acid, comprising the polynucleotide coding sequence of the ET_{A}R antibody provided herein.

Provided herein is a recombinant expression vector, comprising the nucleic acid provided herein.

Provided herein is a host cell, comprising the vector provided herein.

Provided herein is a method for producing an ET_{A}R antibody, comprising culturing the host cell provided herein under conditions that allow the expression of the antibody provided herein.

### Brief Description of the Drawings

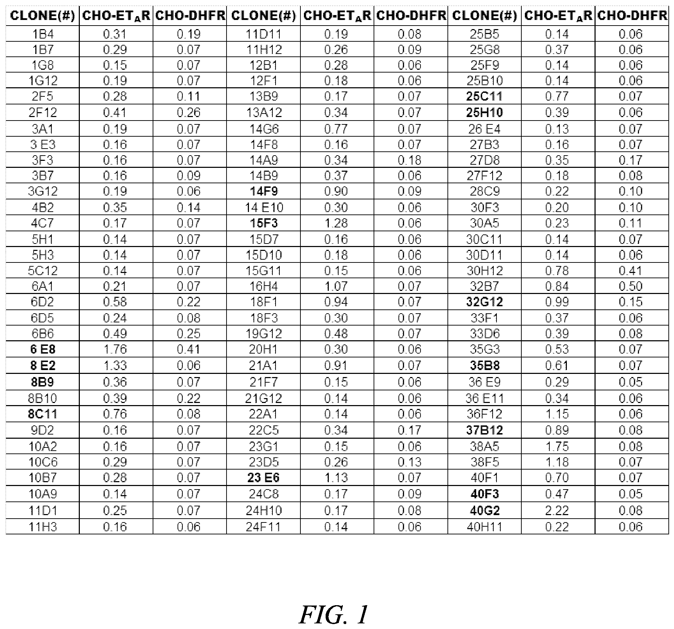

FIG. 1: shows the results of binding of different hybridoma cell supernatants to CHO-DHFR-ET_{A}R (marked as CHO-ET_{A}R in the figure) cells as screened by ELISA, wherein ET_{A}R antibody A-1 (comprising SEQ ID NO: 138 and SEQ ID NO: 166) was cloned from 15F3 hybridoma cells.

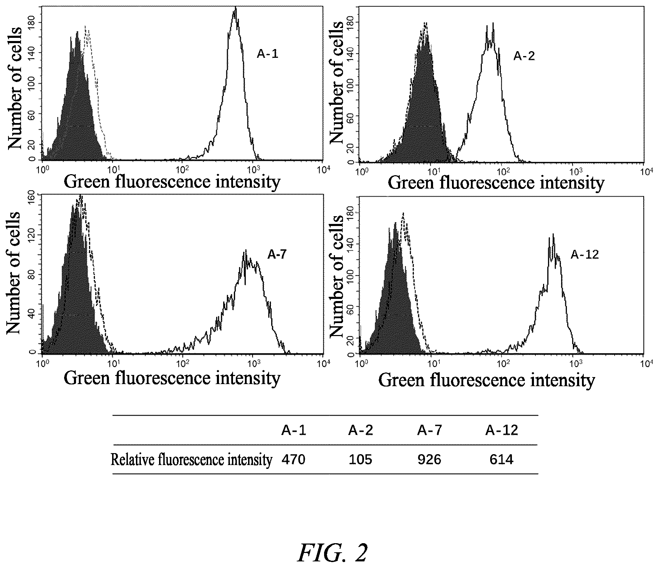

FIG. 2: shows the results of specific binding of recombinantly expressed ET_{A}R antibodies (A-1, A-2 (comprising SEQ ID NO: 140 and SEQ ID NO: 168), A-7 (comprising SEQ ID NO: 150 and SEQ ID NO: 178), and A-12 (comprising SEQ ID NO: 160 and SEQ ID NO: 188)) to human ET_{A}R as detected by fluorescence-activated cell sorting (FACS), wherein the gray peaks and dotted line peaks were negative controls, the dotted line peaks represented curves of binding of the ET_{A}R antibody to CHO-DHFR-, and the solid peaks represented curves of binding of the ET_{A}R antibody to CHO-DHFR-ET_{A}R.

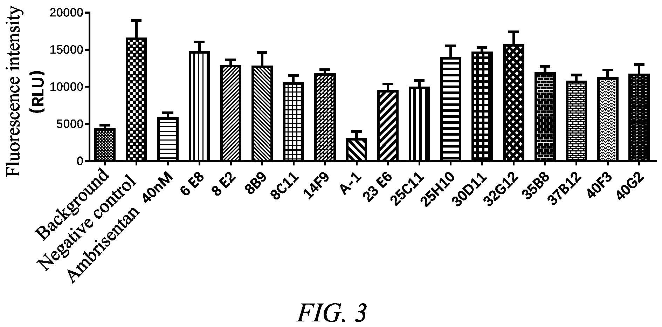

FIG. 3: shows the results of different hybridoma cell supernatants inhibiting intracellular human ET_{A}R mediated Ca²⁺ change as screened by calcium flux experiment.

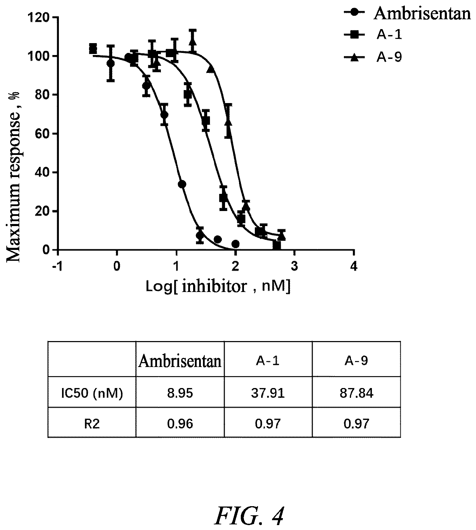

FIG. 4: shows the concentration gradient inhibition curve (IC₅₀ = 38 nM, R² = 0.97) (A-1), (IC₅₀ = 88 nM, R² = 0.97) (A-9 (comprising SEQ ID NO: 154 and SEQ ID NO: 182)) of recombinantly expressed ET_{A}R antibodies and human ET_{A}R by calcium flux detection.
FIG. 5: shows the concentration gradient inhibition curves of antibody A-1 and rabbit ET_{A}R, golden hamster ET_{A}R and rat ET_{A}R by calcium flux detection
FIG. 6: shows the change curves of urea nitrogen (BUN) of animals in the efficacy experiment with antibody A-1 in the rabbit model of acute kidney injury
FIG. 7: shows the change curves of serum creatinine (sCr) of animals in the efficacy experiment with antibody A-1 in the rabbit model of acute kidney injury
FIG. 8: shows the 24-h urinary albumin (U-Alb) excretion of animals in the efficacy experiment with antibody A-1 in the rabbit model of acute kidney injury
FIG. 9: shows the 24-h urinary total protein (U-TP) excretion of animals in the efficacy experiment with antibody A-1 in the golden hamster model of diabetic nephropathy
FIG. 10: shows the 24-h urinary albumin (U-Alb) excretion of animals in the efficacy experiment with antibody A-1 in the golden hamster model of diabetic nephropathy
FIG. 11: shows the 24-h urine volume of animals in the efficacy experiment with antibody A-1 in the golden hamster model of diabetic nephropathy

### Detailed Description of Embodiments

### Definitions

Unless defined otherwise herein, scientific and technical terms shall have the meanings understood by one of ordinary skill in the art. Generally, the nomenclature and techniques related to pharmacology, biology, biochemistry, cell and tissue culture, biology, molecular biology, immunology, microbiology, genetics and protein nucleic acid chemistry as well as hybridization as described herein are well known and commonly used in the art.

Standard one-letter or three-letter abbreviations are used herein to indicate polynucleotide and polypeptide sequences. Unless otherwise indicated, the amino terminus of the polypeptide sequence is on the left and the carboxyl terminus thereof is on the right, and the 5' ends of the upstream chains of single-stranded and double-stranded nucleic acid sequences are on the left and the 3' ends thereof are on the right. The specific portion of a polypeptide can be represented by an amino acid residue number, such as amino acid 80 to amino acid 130, or represented by the actual residue of the site, such as Lys80 to Lys130. The specific polypeptide or polynucleotide sequence can also be described by explaining the difference thereof from the reference sequence.

The terms "peptide", "polypeptide" and "protein" each refer to a molecule comprising two or more amino acids that are interlinked by a peptide bond. These terms cover, for example, natural and artificial proteins, polypeptide analogs of protein fragments and protein sequences (such as mutant proteins, variants and fusion proteins), and proteins that are post-transcriptionally or otherwise covalently or non-covalently modified. A peptide, polypeptide or protein can be monomeric or polymeric.

The term "polypeptide fragment" refers to a polypeptide that has an amino terminal and/or a carboxyl terminal deletion as compared to a corresponding full-length protein. A fragment may be, for example, at least 5, 6, 7, 8, 9, 10, 11, 12, 13, 14, 15, 20, 50, 70, 80, 90, 100, 150 or 200 amino acids in length. A fragment may be, for example, at most 1000, 750, 500, 250, 200, 175, 150, 125, 100, 90, 80, 70, 60, 50, 40, 30, 20, 15, 14, 13, 12, 11 or 10 amino acids in length. A fragment may further comprise, at either or both of its ends, one or more additional amino acids, for example, sequences of amino acids from different natural proteins (e.g., an Fc or a leucine zipper domain) or an artificial amino acid sequence (e.g., an artificial linker sequence).

Polypeptides herein include polypeptides that are modified in any way and for any reason, for example, to: (1) reduce susceptibility to proteolysis, (2) reduce susceptibility to oxidation, (3) alter the affinity for forming protein complexes, (4) alter the binding affinity, and (4) confer or modify other physiochemical or functional properties. Analogs comprise mutant proteins of polypeptides. For example, single or multiple amino acid substitutions (e.g., conservative amino acid substitutions) may be performed in natural sequences (e.g., in the portion of the polypeptide outside the domains that form intramolecular contacts). The "conservative amino acid substitution" is one that does not significantly alter the structural characteristics of a parent sequence (e.g., the substitution of amino acids shall not destroy a helix present in the parent sequence or interfere with other types of secondary structure that characterize the parent sequence or that are necessary for its functionality).

A "variant" of a polypeptide comprises an amino acid sequence in which one or more amino acid residues are inserted into, deleted from, and/or substituted into the amino acid sequence relative to another polypeptide sequence. Variants herein include fusion proteins.

A "derivative" of a polypeptide is a polypeptide that is chemically modified, e.g., by binding to another chemical moiety such as polyethylene glycol and albumin (such as human serum albumin), phosphorylation and glycosylation.

Unless otherwise stated, the term "antibody" includes, in addition to antibodies comprising two full-length heavy chains and two full-length light chains, derivatives, variants, fragments and mutant proteins thereof, and examples of which are listed below.

The term "antibody" is a protein comprising a moiety that binds to an antigen and, optionally, a scaffold or framework moiety that allows the antigen binding moiety to adopt a conformation that promotes the binding of the antibody to the antigen. Examples of antibodies include intact antibodies, antibody fragments (such as antigen binding moieties of antibodies), antibody derivatives, and antibody analogs. The antibody may comprise, for example, an alternative protein scaffold or an artificial scaffold having grafted CDRs or CDR derivatives. The scaffold includes, but is not limited to an antibody-derived scaffold that is introduced, such as one that stabilizes the three-dimensional structure of the antibody, and a fully synthetic scaffold that contains, for example, a biocompatible polymer. See, for example, Korndorfer et al., 2003, Proteins: Structure, Function and Bioinformatics 53:121-129; Roque et al., 2004, Biotechnol. Prog. 20:639-654. In addition, peptide antibody mimetics ("PAMs") and scaffolds based on antibody mimetics using fibronectin as a scaffold may be used.

An antibody may have, for example, the structure of a natural immunoglobulin. The "immunoglobulin" is a tetrameric molecule. In a natural immunoglobulin, each tetramer is composed of two identical pairs of polypeptide chains, each pair having one "light" chain (approximately 25 kDa) and one "heavy" chain (approximately 50-70 kDa). The amino terminal of each chain includes a variable domain of approximately 100 to 110 or more amino acids, which is primarily responsible for antigen recognition. The carboxyl terminal moiety of each chain defines a constant region which is primarily responsible for effector function. Human light chains are classified as κ and λ light chains. Heavy chains are classified as µ, δ, α or ε heavy chains, and define the isotypes of the antigen as IgM, IgD, IgG, IgA and IgE, respectively. Within light and heavy chains, the variable and constant regions are linked by a "J" region of approximately 12 or more amino acids, with the heavy chain also including a "D" region of approximately 10 or more amino acids. See, Fundamental Immunology Ch.7 (edited by Paul, 2nd edition, Raven Press, 1989) (the disclosure of which is incorporated by reference herein in its entirety for any purpose). The variable regions of each light/heavy chain pair form antibody binding sites, such that an intact immunoglobulin has two binding sites.

Natural immunoglobulin chains exhibit the same basic structure of relatively conservative framework regions (FRs) linked by three hypervariable regions, also referred to as complementarity determining regions or CDRs. From N terminus to C terminus, both light and heavy chains comprise domains FR1, CDR1, FR2, CDR2, FR3, CDR3 and FR4. The assignment of amino acids to each domain is in accordance with the definitions of Kabat et al., Sequences of Proteins of Immunological Interest, 5th edition, US Dept. of Health and Human Services, PHS, NIH, NIH Publication No. 91-3242, 1991.

Unless otherwise specified, the "antibody" refers to an intact immunoglobulin or an antigen binding moiety thereof that may compete with an intact antibody for specific binding. Antigen binding moieties may be produced by recombinant DNA techniques or by enzymatic or chemical cleavage of intact antibodies. Antigen binding moieties include, in particular, Fab, Fab', F(ab')₂, Fv, domain antibodies (dAbs), fragments including complementarity determining regions (CDRs), single-chain antibodies (scFv), chimeric antibodies, diabodies, triabodies, tetrabodies, and polypeptides that comprise at least a portion of an immunoglobulin that is sufficient to confer specific antigen binding to the polypeptides.

A Fab fragment is a monovalent fragment having V_{L}, V_{H}, C_{L} and C_{H1} domains; a F(ab')₂ fragment is a divalent fragment having two Fab fragments linked by a disulfide bond at the hinge region; a Fd fragment has a V_{H} or V_{L} domain; and a dAb fragment has a V_{H} domain, a V_{L} domain, or an antigen binding fragment of a V_{H} or V_{L} domain (U.S. Pat. No. 6846634 and 6696245, U.S. Patent Application Publication No. 05/0202512, 04/0202995, 04/0038291, 04/0009507, and 03/0039958, and Ward et al., 1989, Nature 341:544-546.).

A single-chain antibody (scFv) is an antibody in which V_{L} and V_{H} regions are linked by a linker (for example, a synthetic sequence of amino acid residues) to form a continuous protein, wherein the linker is long enough to allow the protein chain to fold back on itself and form a monovalent antigen binding site (see, such as Bird et al., 1988, Science 242:423-26 and Huston et al., 1988, Proc. Natl. Acad. Sci. USA 85:5879-83). A diabody is a divalent antibody comprising two polypeptide chains, wherein each polypeptide chain comprises V_{H} and V_{L} domains linked by a linker that is too short to allow for pairing of the two domains on the same chain, thus allowing each domain to pair with a complementary domain on another polypeptide chain (see, for example, Holliger et al., 1993, Proc. Natl. Acad. Sci. USA 90:6444-48, and Poljak et al., 1994, Structure 2:1121-23). If the two polypeptide chains of a diabody are identical, then a diabody resulting from their pairing will have identical antigen binding sites. Polypeptide chains having different sequences can be used to prepare a diabody having different antigen binding sites. Similarly, triabodies and tetrabodies are antibodies that comprise three and four polypeptide chains, respectively, and form three and four antigen binding sites, respectively, which may be identical or different.

Complementarity determining regions (CDRs) and framework regions (FRs) of a given antibody may be identified using the method described in Kabat et al., Sequences of Proteins of Immunological Interest, 5th edition, US Dept. of Health and Human Services, PHS, NIH, NIH Publication No. 91-3242, 1991. One or more CDRs may be incorporated into a molecule either covalently or noncovalently to make it an antibody. An antibody may incorporate CDR(s) as part of a larger polypeptide chain, may covalently link CDR(s) to another polypeptide chain, or may incorporate CDR(s) noncovalently. CDRs allow for specific binding of an antibody to a specific associated antigen.

Antibodies may have one or more binding sites. If there is more than one binding site, the binding site may be identical to or different from one another. For example, a natural human immunoglobulin usually has two identical binding sites, while a "bispecific" or "bifunctional" antibody has two different binding sites.

The term "murine antibody" includes all antibodies that have one or more variable and constant regions derived from mouse immunoglobulin sequences.

The term "humanized antibody" is an antibody made by grafting the complementarity determining region sequences of a mouse antibody molecule into the framework of human antibody variable regions.

The term "antigen binding domain", "antigen binding region" or "antigen binding site" is a portion of an antibody that comprises amino acid residues (or other moieties) that interact with an antigen and contributes to the specificity and affinity of the antibody to the antigen. For an antibody that specifically binds to an antigen thereof, this will include at least portion of at least one of its CDR domains.

The term "epitope" is a portion of a molecule that is bound by an antibody (for example, by an antibody). An epitope may comprise non-contiguous portions of a molecule (for example, in a polypeptide, amino acid residues that are not contiguous in the primary sequence of the polypeptide but that, in the tertiary and quaternary structures of the polypeptide, are near enough to be bound by an antibody).

The "percent identity" of two polynucleotide or two polypeptide sequences is determined by comparing the sequences using the GAP computer program (a part of the GCG Wisconsin Package, version 10.3 (Accelrys, San Diego, CA)) using its default parameters.

The terms "polynucleotide", "oligonucleotide" and "nucleic acid" are used interchangeably throughout the full text and include DNA molecules (e.g., cDNA or genomic DNA), RNA molecules (e.g., mRNA), DNA or RNA analogs and hybrids thereof produced using nucleotide analogs (e.g., peptide nucleic acids and non-natural nucleotide analogs). Nucleic acid molecules may be single- or double-stranded. In one embodiment, the nucleic acid molecule herein comprises a contiguous open reading frame encoding the antibody or the fragment, derivative, mutant protein or variant thereof provided herein.

If the sequences of two single-stranded polynucleotides are "complementary" to each other, the two single-stranded polynucleotides can be aligned in an antiparallel orientation, so that every nucleotide in one polynucleotide is opposite to its complementary nucleotide in another polynucleotide, without the introduction of gaps, and without unpaired nucleotides at the 5' or 3' end of either sequence. If the two polynucleotides can hybridize to each other under moderately stringent conditions, one polynucleotide is "complementary" to another polynucleotide. Thus, one polynucleotide may be complementary to another polynucleotide, but not the complementary sequence thereof.

The term "vector" is a nucleic acid that can be used to introduce another nucleic acid linked thereto into a cell. One type of vector is a "plasmid", which refers to a linear or circular double-stranded DNA molecule to which additional nucleic acid segments can be linked. Another type of vector is a viral vector (e.g., replication-defective retroviruses, adenoviruses and adenovirus-associated viruses) in which additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell when introduced into the host cell, and thus are replicated along with the host genome. The "expression vector" is a type of vector that can direct the expression of a selected polynucleotide.

If a regulatory sequence affects the expression (e.g., the level, timing, or location of expression) of a nucleotide sequence, the nucleotide sequence is "operably linked" to the regulatory sequence. The "regulatory sequence" is a nucleic acid that affects the expression (e.g., the level, timing, or location of expression) of a nucleic acid to which it is operably linked. A regulatory gene, for example, act directly on a regulated nucleic acid or through one or more other molecules (e.g., polynucleotides that bind to a regulatory sequence and/or nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology, Volume 185, Academic Press, San Diego, CA and Baron et al., 1995, Nucleic Acids Res. 23:3605-06.

The term "host cell" is a cell that can be used to express a nucleic acid such as the nucleic acid provided herein. The host cell may be a prokaryote, for example, E. coli, or it can be eukaryote, such as a unicellular eukaryote (e.g., a yeast or other fungi), a plant cell (e.g., a tobacco or tomato plant cell), an animal cell (e.g., a human cell, a monkey cell, a hamster cell, a rat cell, a mouse cell or an insect cell) or a hybridoma. Generally, a host cell is a culture cell that can be transformed or transfected with a polypeptide-encoding nucleic acid, which can then be expressed in the host cell. The phrase "recombinant host cell" can be used to denote a host cell that is transformed or transfected with a nucleic acid to be expressed. A host cell may also be a cell that comprises the nucleic acid but does not express it at a desired level, unless a regulatory sequence is introduced into the host cell so that it is operably linked to the nucleic acid. It should be understood that the term "host cell" refers not only to the specific subject cell but also to the progeny or possible progeny of such a cell. Because certain modifications may occur in succeeding generations due to, e.g., mutations or environmental influence, such progeny may not, in fact, be identical to the parent cell, but still fall within the scope of the term as used herein.

### Endothelin and endothelin receptor

Endothelin is a polypeptide consisting of 21 amino acids with a molecular weight of 2,400 Da, the N terminus comprises cysteines at positions 1-15 and 3-11 which are linked by two disulfide bonds, and the C terminus comprises some hydrophobic amino acid residues. The N-terminal structure determines the affinity of endothelin to a receptor, while the C-terminal structure determines the position where endothelin binds to the receptor. There are three subtypes of endothelin (ET), namely ET-1, ET-2, and ET-3, which differ in the residues of individual amino acids. Endothelin receptor (ET_{A}R) belongs to subfamily A of the 7-transmembrane receptor family, which is coupled to one or more intracellular signaling pathways through a heterotrimeric guanine nucleotide binding protein (G protein) (Jelinek et al., 1993, Science 259:1614-1616; Segre et al., 1993, Trends Endocrinol. Metab. 4:309-314). As used herein, "endothelin receptor" and "ET_{A}R" are used interchangeably.

In one embodiment, the antibody provided herein can be selected to bind to a membrane-bound endothelin receptor expressed on a cell, and inhibit or block endothelin signaling through the endothelin receptor. In one embodiment, the antibody provided herein specifically binds to a human endothelin receptor. In a further embodiment, the antibody that binds to a human endothelin receptor may also bind to endothelin receptors of other species, such as rats. The examples below provide for the generation of murine antibodies that bind to human membrane-bound endothelin receptors, and in a further embodiment, that bind to endothelin receptors of other species.

The polynucleotide and polypeptide sequences of endothelin receptors of several species are known. SEQ ID NO: 1-SEQ ID NO: 6 show the sequences of Homo sapiens, Rhesus macaque and Rattus norvegicus. The sequence data comes from the GeneBank database of the US National Center for Biotechnology Information.

Endothelin receptor A (ET_{A}R) is as follows:
Homo sapiens polynucleotide (SEQ ID NO: 1); accession number: S63938.
Homo sapiens amino acid (SEQ ID NO: 2); accession number: AAB20278.
Rhesus macaque polynucleotide (SEQ ID NO: 3); accession number: JV635771.
Rhesus macaque amino acid (SEQ ID NO: 4); accession number: AFJ71111.
Rattus norvegicus polynucleotide (SEQ ID NO: 5); accession number: M60786.
Rattus norvegicus amino acid (SEQ ID NO: 6); accession number: AAA41114.
Endothelin receptor A (ET_{A}R) antibody

In one embodiment, provided herein is an ET_{A}R antibody (such as an intact antibody, an antibody fragment, an antibody derivative, a mutant protein of an antibody, and an antibody variant).

In one embodiment, the ET_{A}R antibody provided herein comprises one, two, three, four, five or six amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30;
b. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48;
c. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68;
d. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90;
e. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114; and
f. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

Table 1 lists the light chain CDR amino acid sequences of the ET_{A}R antibody provided herein, and the corresponding polynucleotide coding sequences thereof. Table 2 lists the heavy chain CDR amino acid sequences of the ET_{A}R antibody provided herein, and the corresponding polynucleotide coding sequences thereof.

**Table 1: Light chain CDR amino acid sequences and polynucleotide coding sequences thereof**

| | CDR1 | CDR2 | CDR3 |
|---|---|---|---|
| A-1 nucleotide | agggccagtcagaacattgg cacaagcatacac (SEQ ID NO: 7) | tatgcttctaagtctatatct (SEQ ID NO: 31) | caacatagttatagctggcc gtggacg (SEQ ID NO: 49) |
| A-1 amino acid | RASQNIGTSIH (SEQ ID NO: 8) | YASKSIS (SEQ ID NO: 32) | QHSYSWPWT (SEQ ID NO: 50) |
| A-2 nucleotide | cgagcaagtgaaaatatttac agttatttagca (SEQ ID NO: 9) | aatgcaaaaaccttagcagaa (SEQ ID NO: 33) | cagcatcattatggtattccgt tcacg (SEQ ID NO: 51) |
| A-2 amino acid | RASENIYSYLA (SEQ ID NO: 10) | NAKTLAE (SEQ ID NO: 34) | QHHYGIPFT (SEQ ID NO: 52) |
| A-3 nucleotide | cagagcctctttgatattgatg gaaagacatatttgaat (SEQ ID NO: 11) | ctggtgtctgaattggactct (SEQ ID NO:35) | tggcaaggtacacattttccg ctcacg (SEQ ID NO: 53) |
| A-3 amino acid | QSLFDIDGKTYLN (SEQ ID NO: 12) | LVSELDS (SEQ ID NO: 36) | WQGTHFPLT (SEQ ID NO: 54) |
| A-4 nucleotide | cgggcaagtcaggacattggt ggtagcttaaac (SEQ ID NO: 13) | gccacatccagcttagattct (SEQ ID NO: 37) | ctacaatatgctagttctccgt atacg (SEQ ID NO: 55) |
| A-4 amino acid | RASQDIGGSLN (SEQ ID NO: 14) | ATSSLDS (SEQ ID NO: 38) | LQYASSPYT (SEQ ID NO: 56) |
| A-5 nucleotide | agggccagccagactattag cgacttcttacac (SEQ ID NO: 15) | tatgcttcccaatccatctct (SEQ ID NO: 39) | caaagtggtaacacctttccg tggacg (SEQ ID NO: 57) |
| A-5 amino acid | RASQTISDFLH (SEQ ID NO: 16) | YASQSIS (SEQ ID NO: 40) | QSGNTFPWT (SEQ ID NO: 58) |
| A-6 nucleotide | agggcaagtgaggacataca cactcaattagcc (SEQ ID NO: 17) | ggtgcagccagtttgaaaagt (SEQ ID NO: 41) | caacagtataggagtattcc gtggacg (SEQ ID NO: 59) |
| A-6 amino acid | RASEDIHTQLA (SEQ ID NO: 18) | GAASLKS (SEQ ID NO: 42) | QQYRSIPWT (SEQ ID NO: 60) |
| A-7 nucleotide | agatctagtcagtacattgttca tagtactggaaccacctattta gaa (SEQ ID NO: 19) | aaagtttccaaccgattttct (SEQ ID NO: 43) | tttcaaggttcacattttccatt cacg (SEQ ID NO: 61) |
| A-7 amino acid | RSSQYIVHSTGTTY LE (SEQ ID NO: 20) | KVSNRFS (SEQ ID NO: 44) | FQGSHFPFT (SEQ ID NO: 62) |
| A-8 nucleotide | agatctagtcattaccttgttca tgataacggaaacacctatgtt gaa (SEQ ID NO: 21) | aaggtttccaaccgattttct (SEQ ID NO: 43) | tttcaaggttcacatttcccatt cacg (SEQ ID NO: 63) |
| A-8 amino acid | RSSHYLVHDNGNT YVE (SEQ ID NO: 22) | KVSNRFS (SEQ ID NO: 44) | FQGSHFPFT (SEQ ID NO: 62) |
| A-9 nucleotide | agatctagtcagaacattgtcc atagtactggaaacacctattt agaa (SEQ ID NO: 23) | aaagtttccaaccgattttct (SEQ ID NO: 43) | tttcaaggttcacattttccatt cacg (SEQ ID NO: 61) |
| A-9 amino acid | RSSQNIVHSTGNTY LE (SEQ ID NO: 24) | KVSNRFS (SEQ ID NO: 44) | FQGSHFPFT (SEQ ID NO: 62) |
| A-10 nucleotide | agtgtcagctcaagtgtaagtt acatacac (SEQ ID NO: 25) | gacacatccaaactggcttct (SEQ ID NO: 45) | caccagtggagtactaaccc acccacg (SEQ ID NO: 63) |
| A-10 amino acid | SVSSSVSYIH (SEQ ID NO: 26) | DTSKLAS (SEQ ID NO: 46) | HQWSTNPPT (SEQ ID NO: 64) |
| A-11 nucleotide | agtgccagctcaagtgtaagtt acatgtgc (SEQ ID NO: 27) | gacacatccaaactggcttct (SEQ ID NO: 45) | cagcagtggagtagtaaccc acccacg (SEQ ID NO: 65) |
| A-11 amino acid | SASSSVSYMC (SEQ ID NO: 28) | DTSKLAS (SEQ ID NO: 46) | QQWSSNPPT (SEQ ID NO: 66) |
| A-12 nucleotide | cagggcattaacaattat (SEQ ID NO: 29) | tatacatcaactttacagtca (SEQ ID NO: 47) | cagcagtttagtaaacttcgg aca (SEQ ID NO: 67) |
| A-12 amino acid | QGINNY (SEQ ID NO: 30) | YTSTLQS (SEQ ID NO: 48) | QQFSKLRT (SEQ ID NO: 68) |

**Table 2: Heavy chain CDR amino acid sequences and polynucleotide coding sequences thereof**

| | CDR1 | CDR2 | CDR3 |
|---|---|---|---|
| A-1 nucleotide | gggttctcactgaccacttct ggcttgggtgttgcc (SEQ ID NO: 69) | cacatttggtcggatggtgac acgcgctattacccagccctg aagaac (SEQ ID NO: 91) | atgaaggatgatagtctttacttt gacaac (SEQ ID NO:115) |
| A-1 amino acid | GFSLTTSGLGVA (SEQ ID NO: 70) | HIWSDGDTRYYPA LKN (SEQ ID NO: 92) | MKDDSLYFDN (SEQ ID NO: 116) |
| A-2 nucleotide | ggctacacctttactagctac tggatacac (SEQ ID NO: 71) | tacattaatcctgacactgatta tagtgagtacaat (SEQ ID NO:93) | gcaagtgctggttattatttttttg acttc (SEQ ID NO: 117) |
| A-2 amino acid | GYTFTSYWIH (SEQ ID NO:72) | YINPDTDYSEYN (SEQ ID NO: 94) | ASAGYYFFDF (SEQ ID NO: 118 |
| A-3 nucleotide | ggcctcaacattaaagacat ctatattcac (SEQ ID NO: 73) | aggattgatcctgcgaacggt aagactgcatatgac (SEQ ID NO: 95) | ggtaggggggcccac (SEQ ID NO:119) |
| A-3 amino acid | GLNIKDIYIH (SEQ ID NO:74) | RIDPANGKTAYD (SEQ ID NO: 96) | GRGAH (SEQ ID NO:120) |
| A-4 nucleotide | ggttactcattcaccaactac tggatacac (SEQ ID NO: 75) | atgattgatccttccgatgctg aaactgggttaaat (SEQ ID NO:97) | gcaagaattggcgattactata atatggactac (SEQ ID NO:121) |
| A-4 amino acid | GYSFTNYWIH (SEQ ID NO: 76) | MIDPSDAETGLN (SEQ ID NO: 98) | ARIGDYYNMDY (SEQ ID NO:122) |
| A-5 nucleotide | ggattcactttcagtgactat cccatgtct (SEQ ID NO: 77) | gttagtgatggtggtggttcca cc (SEQ ID NO: 99) | acaagacatgcttcctactata gctacgaccattctatggacta c (SEQ ID NO:123) |
| A-5 amino acid | GFTFSDYPMS (SEQ ID NO: 78) | VSDGGGST (SEQ ID NO: 100) | TRHASYYSYDHSM DY (SEQ ID NO:124) |
| A-6 nucleotide | ggattcactttcagtagcttt ggcatgtct (SEQ ID NO: 79) | attagtagtgctggtagtttcac c (SEQ ID NO: 101) | gcaagacgggggtacgacgtt gggtgctttgaccac (SEQ ID NO:125) |
| A-6 amino acid | GFTFSSFGMS (SEQ ID NO: 80) | ISSAGSFT (SEQ ID NO: 102) | ARRGYDVGCFDH (SEQ ID NO: 126) |
| A-7 nucleotide | ggattcactttcagtacctat ggcatgtct (SEQ ID NO: 81) | accattaatactaatggtggta ccacctattatcgagacagtgt gaagggc (SEQ ID NO: 103) | gcaagagactacggggctatg gactac (SEQ ID NO: 127) |
| A-7 amino acid | GFTFSTYGMS (SEQ ID NO: 82) | TINTNGGTTYYRDS VKG (SEQ ID NO: 104) | ARDYGAMDY (SEQ ID NO: 128) |
| A-8 nucleotide | ggattcactttcagtacctat ggcatgtct (SEQ ID NO: 81) | accataaatactaatggtggta acacctattattcagacaatgt gaagggc (SEQ ID NO: 105) | gcaagagactacggggctatg gactac (SEQ ID NO: 127) |
| A-8 amino acid | GFTFSTYGMS (SEQ ID NO:82) | TINTNGGNTYYSDN VKG (SEQ ID NO: 106) | ARDYGAMDY (SEQ ID NO: 128) |
| A-9 nucleotide | ggattcactttcagtagttat ggcatgtct (SEQ ID NO: 83) | accattagtactaatggtgcca ccgccaattatccagacagtg tgaagggc (SEQ ID NO:107) | gcaactgaaaagggagctatg ggctac (SEQ ID NO:129) |
| A-9 amino acid | GFTFSSYGMS (SEQ ID NO: 84) | TISTNGATANYPDS VKG (SEQ ID NO: 108) | ATEKGAMGY (SEQ ID NO: 130) |
| A-10 nucleotide | gggttttcactgaccacttct ggtatgggtgtaggc (SEQ ID NO: 85) | cacatttggtgggatgatgata agtactataatccatccctgaa gagc (SEQ ID NO: 109) | gctcgaagaactgagactatg attacgacagtgctatattactat gctatggactac (SEQ ID NO:131) |
| A-10 amino acid | GFSLTTSGMGVG (SEQ ID NO: 86) | HIWWDDDKYYNPS LKS (SEQ ID NO: 110) | ARRTETMITTVLYY YAMDY (SEQ ID NO: 132) |
| A-11 nucleotide | ggattttcactgagcacttct ggtttgggtgtaggc (SEQ ID NO:87) | cacatttggtgggatgatgata agtactataatccatcccttaa gaga (SEQ ID NO: 111) | gctcgaaggagggaagttaac ttcggtattaactattactattcta tggactac (SEQ ID NO: 133) |
| A-11 amino acid | GFSLSTSGLGVG (SEQ ID NO: 88) | HIWWDDDKYYNPS LKR (SEQ ID NO: 112) | ARRREVNFGINYYY SMDY (SEQ ID NO: 134) |
| A-12 nucleotide | ggattcaccttcagtgattatt ac (SEQ ID NO: 89) | attagaaatcgggctaatggtt acacaaca (SEQ ID NO: 113) | gtaagagattcctatcactacg ggtacttcgatgtc (SEQ ID NO: 135) |
| A-12 amino acid | GFTFSDYY (SEQ ID NO:90) | IRNRANGYTT (SEQ ID NO:114) | VRDSYHYGYFDV (SEQ ID NO: 136) |

In one embodiment, the antibody provided herein comprises a sequence that differs from each of the CDR amino acid sequences listed in Tables 1 and 2 by 5, 4, 3, 2 or 1 single amino acid addition, substitution and/or deletion. In another embodiment, the antibody provided herein comprises a sequence that differs from each of the CDR amino acid sequences listed in Tables 1 and 2 by 4, 3, 2 or 1 single amino acid addition, substitution and/or deletion. In another embodiment, the antibody provided herein comprises a sequence that differs from each of the CDR amino acid sequences listed in Tables 1 and 2 by 3, 2 or 1 single amino acid addition, substitution and/or deletion. In another embodiment, the antibody provided herein comprises a sequence that differs from each of the CDR amino acid sequences listed in Tables 1 and 2 by 2 or 1 single amino acid addition, substitution and/or deletion. In another embodiment, the antibody provided herein comprises a sequence that differs from each of the CDR amino acid sequences listed in Tables 1 and 2 by 1 single amino acid addition, substitution and/or deletion.

In another embodiment, the ET_{A}R antibody (ET_{A}R-1 antibody) provided herein comprises one or two amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30; and
b. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90.

In one aspect, the ET_{A}R-1 antibody further comprises one or two amino acid sequences, wherein each amino acid sequence is independently selected from the amino acid sequences listed below:
a. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48; and
b. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114.

In another aspect, the ET_{A}R-1 antibody further comprises one or two amino acid sequences, wherein each amino acid sequence is independently selected from the amino acid sequences listed below:
a. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68; and
b. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

In another embodiment, the ET_{A}R antibody (ET_{A}R-2 antibody) provided herein comprises one or two amino acid sequences, wherein each amino acid sequence is independently selected from the amino acid sequences listed below:
a. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48; and
b. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114.

In one aspect, the ET_{A}R-2 antibody further comprises one or two amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30; and
b. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90.

In another aspect, the ET_{A}R-2 antibody further comprises one or two amino acid sequences, wherein each amino acid sequence is independently selected from the amino acid sequences listed below:
a. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68; and
b. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

In another embodiment, the ET_{A}R antibody (ET_{A}R-3 antibody) provided herein comprises one or two amino acid sequences, wherein each amino acid sequence is independently selected from the amino acid sequences listed below:
a. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68; and
b. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

In one aspect, the ET_{A}R-3 antibody further comprises one or two amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30; and
b. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90.

In another aspect, the ET_{A}R-3 antibody further comprises one or two amino acid sequences, wherein each amino acid sequence is independently selected from the amino acid sequences listed below:
a. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48; and
b. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114.

In one embodiment, the ET_{A}R antibody provided herein comprises:
a. a light chain CDR1 amino acid sequence independently selected from the list below: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30;
b. a light chain CDR2 amino acid sequence independently selected from the list below: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48;
c. a light chain CDR3 amino acid sequence independently selected from the list below: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68;
d. a heavy chain CDR1 amino acid sequence independently selected from the list below: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90;
e. a heavy chain CDR2 amino acid sequence independently selected from the list below: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114; and
f. a heavy chain CDR3 amino acid sequence independently selected from the list below: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

In one embodiment, the ET_{A}R antibody provided herein comprises a light chain CDR3 amino acid sequence independently selected from the list below: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68. In another embodiment, the ET_{A}R antibody provided herein comprises a heavy chain CDR3 amino acid sequence independently selected from the list below: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136. In another embodiment, the ET_{A}R antibody provided herein comprises a combination of light chain and heavy chain CDR3 amino acid sequences independently selected from the list below: SEQ ID NO: 50 and SEQ ID NO: 116, SEQ ID NO: 62 and SEQ ID NO: 128, SEQ ID NO: 62 and SEQ ID NO: 130, SEQ ID NO: 64 and SEQ ID NO: 132, SEQ ID NO: 66 and SEQ ID NO: 134, and SEQ ID NO: 68 and SEQ ID NO: 136.

In another embodiment, the ET_{A}R antibody provided herein comprises one or two amino acid sequences, wherein each amino acid sequence is independently selected from the amino acid sequences listed below:
a. light chain variable domain amino acid sequences: SEQ ID NO: 138 (L1), SEQ ID NO: 140 (L2), SEQ ID NO: 142 (L3), SEQ ID NO: 144 (L4), SEQ ID NO: 146 (L5), SEQ ID NO: 148 (L6), SEQ ID NO: 150 (L7), SEQ ID NO: 152 (L8), SEQ ID NO: 154 (L9), SEQ ID NO: 156 (L10), SEQ ID NO: 158 (L11), SEQ ID NO: 160 (L12), SEQ ID NO: 162 (L13), and SEQ ID NO: 164 (L14), and an amino acid sequence having at least 80%, at least 85%, at least 90%, or at least 95% identity thereto; and
b. heavy chain variable domain amino acid sequences: SEQ ID NO: 166 (H1), SEQ ID NO: 168 (H2), SEQ ID NO: 170 (H3), SEQ ID NO: 172 (H4), SEQ ID NO: 174 (H5), SEQ ID NO: 176 (H6), SEQ ID NO: 178 (H7), SEQ ID NO: 180 (H8), SEQ ID NO: 182 (H9), SEQ ID NO: 184 (H10), SEQ ID NO: 186 (H11), SEQ ID NO: 188 (H12), SEQ ID NO: 190 (H13), and SEQ ID NO: 192 (H14), and an amino acid sequence having least 80%, at least 85%, at least 90%, or at least 95% identity thereto.

In another embodiment, the polynucleotide coding sequence of the ET_{A}R antibody provided herein comprises one or two polynucleotide sequences, wherein each polynucleotide sequence is independently selected from the polynucleotide sequences listed below:
a. light chain variable domain polynucleotide coding sequences: SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, and SEQ ID NO: 163, and a polynucleotide sequence having at least 80%, at least 85%, at least 90%, or at least 95% identity thereto; and
b. heavy chain variable domain polynucleotide coding sequences: SEQ ID NO: 165, SEQ ID NO: 167, SEQ ID NO: 169, SEQ ID NO: 171, SEQ ID NO: 173, SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 189, and SEQ ID NO: 191, and a polynucleotide sequence having at least 80%, at least 85%, at least 90%, or at least 95% identity thereto.

In another embodiment, the ET_{A}R antibody provided herein comprises:
a. a light chain variable domain amino acid sequence independently selected from the list below: SEQ ID NO: 138 (L1), SEQ ID NO: 140 (L2), SEQ ID NO: 142 (L3), SEQ ID NO: 144 (L4), SEQ ID NO: 146 (L5), SEQ ID NO: 148 (L6), SEQ ID NO: 150 (L7), SEQ ID NO: 152 (L8), SEQ ID NO: 154 (L9), SEQ ID NO: 156 (L10), SEQ ID NO: 158 (L11), SEQ ID NO: 160 (L12), SEQ ID NO: 162 (L13), and SEQ ID NO: 164 (L14), and an amino acid sequence having at least 80%, at least 85%, at least 90%, or at least 95% identity thereto; and
b. a heavy chain variable domain amino acid sequence independently selected from the list below: SEQ ID NO: 166 (H1), SEQ ID NO: 168 (H2), SEQ ID NO: 170 (H3), SEQ ID NO: 172 (H4), SEQ ID NO: 174 (H5), SEQ ID NO: 176 (H6), SEQ ID NO: 178 (H7), SEQ ID NO: 180 (H8), SEQ ID NO: 182 (H9), SEQ ID NO: 184 (H10), SEQ ID NO: 186 (H11), SEQ ID NO: 188 (H12), SEQ ID NO: 190 (H13), and SEQ ID NO: 192 (H14), and an amino acid sequence having least 80%, at least 85%, at least 90%, or at least 95% identity thereto.

In another embodiment, the ET_{A}R antibody provided herein comprises:
a. a light chain variable domain amino acid sequence independently selected from the list below: SEQ ID NO: 138 (L1), SEQ ID NO: 140 (L2), SEQ ID NO: 142 (L3), SEQ ID NO: 144 (L4), SEQ ID NO: 146 (L5), SEQ ID NO: 148 (L6), SEQ ID NO: 150 (L7), SEQ ID NO: 152 (L8), SEQ ID NO: 154 (L9), SEQ ID NO: 156 (L10), SEQ ID NO: 158 (L11), SEQ ID NO: 160 (L12), SEQ ID NO: 162 (L13), and SEQ ID NO: 164 (L14); and
b. a heavy chain variable domain amino acid sequence independently selected from the list below: SEQ ID NO: 166 (H1), SEQ ID NO: 168 (H2), SEQ ID NO: 170 (H3), SEQ ID NO: 172 (H4), SEQ ID NO: 174 (H5), SEQ ID NO: 176 (H6), SEQ ID NO: 178 (H7), SEQ ID NO: 180 (H8), SEQ ID NO: 182 (H9), SEQ ID NO: 184 (H10), SEQ ID NO: 186 (H11), SEQ ID NO: 188 (H12), SEQ ID NO: 190 (H13), and SEQ ID NO: 192 (H14).

In another embodiment, the ET_{A}R antibody provided herein comprises a combination of light chain and heavy chain variable domain amino acid sequences independently selected from the list below: SEQ ID NO: 138 and SEQ ID NO: 166 (L1H1), SEQ ID NO: 140 and SEQ ID NO: 168 (L2H2), SEQ ID NO: 142 and SEQ ID NO: 170 (L3H3), SEQ ID NO: 144 and SEQ ID NO: 172 (L4H4), SEQ ID NO: 146 and SEQ ID NO: 174 (L5H5), SEQ ID NO: 148 and SEQ ID NO: 176 (L6H6), SEQ ID NO: 150 and SEQ ID NO: 178 (L7H7), SEQ ID NO: 152 and SEQ ID NO: 180 (L8H8), SEQ ID NO: 154 and SEQ ID NO: 182 (L9H9), SEQ ID NO: 156 and SEQ ID NO: 184 (L10H10), SEQ ID NO: 158 and SEQ ID NO: 186 (L11H11), SEQ ID NO: 160 and SEQ ID NO: 188 (L12H12), SEQ ID NO: 162 and SEQ ID NO: 190 (L13H13), and SEQ ID NO: 164 and SEQ ID NO: 192 (L14H14).

The ET_{A}R antibody provided herein may also be represented herein by the "LxHy" notation, where "x" corresponds to the light chain variable region and "y" corresponds to the heavy chain variable region. For example, L2H1 refers to an antibody having a light chain variable region comprising the amino acid sequence SEQ ID NO: 140 (L2) and a heavy chain variable region comprising the amino acid sequence SEQ ID NO: 166 (H1).

In another embodiment, the ET_{A}R antibody provided herein comprises a light chain variable region selected from L1-L14 or a heavy chain variable region selected from H1-H14, and fragments, derivatives, mutant proteins or variants thereof.

In another embodiment, the ET_{A}R antibody provided herein comprises a combination of light chain and heavy chain CDR3 amino acid sequences independently selected from the list below: SEQ ID NO: 138 and SEQ ID NO: 166, SEQ ID NO: 150 and SEQ ID NO: 178, SEQ ID NO: 152 and SEQ ID NO: 180, SEQ ID NO: 154 and SEQ ID NO: 182, SEQ ID NO: 156 and SEQ ID NO: 184, SEQ ID NO: 158 and SEQ ID NO: 186, SEQ ID NO: 160 and SEQ ID NO: 188, SEQ ID NO: 162 and SEQ ID NO: 190, and SEQ ID NO: 164 and SEQ ID NO: 192.

In one embodiment, the ET_{A}R antibody provided herein comprises the light chain variable domain amino acid sequence SEQ ID NO: 138 or the heavy chain variable domain amino acid sequence SEQ ID NO: 166. In another embodiment, the ET_{A}R antibody provided herein comprises the light chain variable domain amino acid sequence SEQ ID NO: 138 and the heavy chain variable domain amino acid sequence SEQ ID NO: 166.

In another embodiment, the ET_{A}R antibody provided herein further comprises constant region amino acid sequences, wherein each constant region amino acid sequence is independently selected from the amino acid sequences listed below:
a. light chain constant region amino acid sequences: SEQ ID NO: 194 and SEQ ID NO: 196; and
b. a heavy chain constant region amino acid sequence: SEQ ID NO: 198.

In another embodiment, the ET_{A}R antibody provided herein further comprises constant region amino acid sequences, wherein each constant region amino acid sequence is independently selected from the combinations of light chain and heavy chain constant region amino acid sequences listed below:
a. a combination of the light chain constant region amino acid sequence SEQ ID NO: 194 and the heavy chain constant region amino acid sequence SEQ ID NO: 198; and
b. a combination of the light chain constant region amino acid sequence SEQ ID NO: 196 and the heavy chain constant region amino acid sequence SEQ ID NO: 198.

In one embodiment, the antibody provided herein comprises light chain and heavy chain CDR and FR (framework) amino acid sequences listed herein. In one embodiment, the antibody comprises the light chain CDR1 sequence listed herein. In another embodiment, the antibody comprises the light chain CDR2 sequence listed herein. In another embodiment, the antibody comprises the light chain CDR3 sequence listed herein. In another embodiment, the antibody comprises the heavy chain CDR1 sequence listed herein. In another embodiment, the antibody comprises the heavy chain CDR2 sequence listed herein. In another embodiment, the antibody comprises the heavy chain CDR3 sequence listed herein. In another embodiment, the antibody comprises the light chain FR1 sequence listed herein. In another embodiment, the antibody comprises the light chain FR2 sequence listed herein. In another embodiment, the antibody comprises the light chain FR3 sequence listed herein. In another embodiment, the antibody comprises the light chain FR4 sequence listed herein. In another embodiment, the antibody comprises the heavy chain FR1 sequence listed herein. In another embodiment, the antibody comprises the heavy chain FR2 sequence listed herein. In another embodiment, the antibody comprises the heavy chain FR3 sequence listed herein. In another embodiment, the antibody comprises the heavy chain FR4 sequence listed herein.

In one embodiment, the CDR3 sequence of the antibody differs from a combination of the light and heavy chain CDR3 amino acid sequences SEQ ID NO: 50 and SEQ ID NO: 116 listed herein by at most 6, 5, 4, 3, 2 or 1 single amino acid addition, substitution and/or deletion. In another embodiment, the light chain CDR3 sequence of the antibody differs from the light chain CDR3 amino acid sequence SEQ ID NO: 50 listed herein by no more than 6, 5, 4, 3, 2 or 1 single amino acid addition, substitution and/or deletion. In another embodiment, the light chain CDR3 sequence of the antibody differs from the light chain CDR3 amino acid sequence SEQ ID NO: 50 listed herein by no more than 6, 5, 4, 3, 2 or 1 single amino acid addition, substitution and/or deletion, and the heavy chain CDR3 sequence of the antibody differs from the heavy chain CDR3 amino acid sequence SEQ ID NO: 116 or SEQ ID NO: 118 listed herein by at most 6, 5, 4, 3, 2 or 1 single amino acid addition, substitution and/or deletion. In another embodiment, the antibody further comprises 1, 2, 3, 4, 5 or 6 combinations of the light and heavy chain CDR sequences listed herein. In another embodiment, the antibody further comprises 1, 2, 3, 4, 5 or 6 combinations of light and heavy chain CDR sequences, and each sequence individually differs from a combination of the light and heavy chain CDR3 amino acid sequences SEQ ID NO: 50 and SEQ ID NO: 116 listed herein by at most 6, 5, 4, 3, 2 or 1 single amino acid. In another embodiment, the antibody comprises the light chain variable region CDRs and the heavy chain variable region CDRs listed herein. In another embodiment, the antibody comprises 1, 2, 3, 4, 5 or 6 combinations of the light and heavy chain CDR sequences listed herein.

In one embodiment, the antibody (e.g., an antibody or antibody fragment) comprises the L1 light chain variable domain sequence listed herein. In one embodiment, the light chain variable domain comprises an amino acid sequence that differs from the L1 light chain variable domain sequence by 15, 14, 13, 12, 11, 10, 9, 8, 7, 6, 5, 4, 3, 2 or 1 amino acid, wherein each of the sequences differs independently by a deletion, insertion or substitution of an amino acid residue. In another embodiment, the light chain variable domain comprises an amino acid sequence having at least 70%, at least 75%, at least 80%, at least 85%, at least 90%, at least 95%, at least 97%, or at least 99% identity to the L1 light chain variable domain sequence. In another embodiment, the light chain variable domain polynucleotide coding sequence comprises a nucleotide coding sequence having at least 70%, at least 75%, at least 80%, at least 85%, at least 90%, at least 95%, at least 97%, or at least 99% identity to the L1 polynucleotide coding sequence. In another embodiment, the light chain variable domain polynucleotide coding sequence comprises a polynucleotide sequence that hybridizes to the complementary sequence of the L1 light chain variable domain polynucleotide coding sequence under moderately stringent conditions. In another embodiment, the light chain variable domain polynucleotide coding sequence comprises a polynucleotide sequence that hybridizes to the complementary sequence of the L1 light chain variable domain polynucleotide coding sequence under stringent conditions.

In one embodiment, the antibody (e.g., an antibody or antibody fragment) comprises the H1 heavy chain variable domain sequence listed herein. In another embodiment, the variable domain comprises an amino acid sequence that differs from the H1 heavy chain variable domain sequence by 15, 14, 13, 12, 11, 10, 9, 8, 7, 6, 5, 4, 3, 2 or 1 amino acid, wherein each of the sequences differs independently by a deletion, insertion or substitution of an amino acid residue. In another embodiment, the heavy chain variable domain comprises an amino acid sequence having at least 70%, at least 75%, at least 80%, at least 85%, at least 90%, at least 95%, at least 97%, or at least 99% identity to the H1 heavy chain variable domain sequence. In another embodiment, the heavy chain variable domain polynucleotide coding sequence comprises a nucleotide coding sequence having at least 70%, at least 75%, at least 80%, at least 85%, at least 90%, at least 95%, at least 97%, or at least 99% identity to the H1 polynucleotide coding sequence. In another embodiment, the heavy chain variable domain polynucleotide coding sequence comprises a polynucleotide sequence that hybridizes to the complementary sequence of the H1 heavy chain variable domain polynucleotide coding sequence under moderately stringent conditions. In one embodiment, the heavy chain variable domain polynucleotide coding sequence comprises a polynucleotide sequence that hybridizes to the complementary sequence of the H1 heavy chain variable domain polynucleotide coding sequence under stringent conditions.

In one embodiment, the antibody provided herein includes an antibody comprising a combination of L1H1, L2H2, L3H3, L4H4, L5H5, L6H6, L7H7, L8H8, L9H9, L10H10, L11H11, L12H12, L13H13, or L14H14, or a desired phenotype thereof (e.g., IgA, IgG1, IgG2a, IgG2b, IgG3, IgM, IgE and IgD), or a Fab or F(ab')₂ fragment thereof.

In one embodiment, the antibody provided herein includes an antibody comprising a combination of L1H1, or a class-switched antibody thereof (e.g., IgA, IgG1, IgG2a, IgG2b, IgG3, IgM, IgE and IgD), or a Fab or F(ab')₂ fragment thereof.

The antibody provided herein (e.g., an antibody, an antibody fragment and an antibody derivative) may comprise any of the constant regions known in the art. The light chain constant region may be, for example, a κ or λ light chain constant region, such as a mouse κ or λ light chain constant region. The heavy chain constant region may be, for example, an α, δ, ε, γ or µ heavy chain constant region, such as a mouse α, δ, ε, γ or µ heavy chain constant region. In one embodiment, the light or heavy chain constant region is a fragment, derivative, variant or mutant protein of a natural constant region.

In one embodiment, the antibody provided herein further comprises a κ or λ light chain constant domain or fragments thereof. The light chain constant region sequences and the polynucleotide coding sequences thereof are provided below:
polynucleotide (κ), (SEQ ID NO: 193); amino acid (κ), (SEQ ID NO: 194);
polynucleotide (λ), (SEQ ID NO: 195); amino acid (λ), (SEQ ID NO: 196).

In another embodiment, the antibody provided herein further comprises a heavy chain constant domain or a fragment thereof. The heavy chain constant region sequences and the polynucleotide coding sequences thereof are provided below:
polynucleotide (IgG1), (SEQ ID NO: 197); amino acid (IgG1), (SEQ ID NO: 198).

In one embodiment, the ET_{A}R antibody provided herein is selected from a murine antibody, a humanized antibody, a chimeric antibody, a monoclonal antibody, a polyclonal antibody, a recombinant antibody, an antigen binding antibody fragment, a single-chain antibody, a diabody, a triabody, a tetrabody, a Fab fragment, a F(ab')ₓ fragment, a domain antibody, an IgD antibody, an IgE antibody, an IgM antibody, an IgGl antibody, an IgG2 antibody, an IgG3 antibody and an IgG4 antibody. In another embodiment, the ET_{A}R antibody provided herein is an ET_{A}R monoclonal antibody. In another embodiment, the ET_{A}R antibody provided herein is a murine ET_{A}R antibody. The ET_{A}R antibody provided herein is a humanized ET_{A}R antibody.

In one embodiment, the ET_{A}R antibody provided herein is a monoclonal antibody A-1 (comprising SEQ ID NO: 138 and SEQ ID NO: 166), A-7 (comprising SEQ ID NO: 150 and SEQ ID NO: 178), A-8 (comprising SEQ ID NO: 152 and SEQ ID NO: 180), A-9 (comprising SEQ ID NO: 154 and SEQ ID NO: 182), A-10 (comprising SEQ ID NO: 156 and SEQ ID NO: 184), A-11 (comprising SEQ ID NO: 158 and SEQ ID NO: 186), A-12 (comprising SEQ ID NO: 160 and SEQ ID NO: 188), A-13 (comprising SEQ ID NO: 162 and SEQ ID NO: 190), or A-14 (comprising SEQ ID NO: 164 and SEQ ID NO: 192).

### Antibody and antibody fragment

In one embodiment, the antibody provided herein is an intact antibody (including polyclonal, monoclonal, chimeric, humanized or human antibodies having full length heavy and/or light chains). In another embodiment, the antibody provided herein is an antibody fragment, for example, a F(ab')2, Fab, Fab', Fv, Fc or Fd fragment, and may be incorporated into single domain antibodies, single-chain antibodies, maxibodies, minibodies, intrabodies, diabodies, triabodies, tetrabodies, v-NAR and bis-scFv (see, for example, Hollinger and Hudson, 2005, Nature Biotechnology 23:1126-1136). In another embodiment, the antibody provided herein also includes antibody polypeptides such as those disclosed in U.S. Pat. No. US 6703199, including fibronectin polypeptide monoclonal antibodies. In another embodiment, the antibody provided herein also includes other antibody polypeptides such as those disclosed in U.S. patent application publication 2005/0238646, which are single-chain polypeptides.

In one embodiment, the variable regions of the genes expressing related monoclonal antibodies in hybridomas are amplified using nucleotide primers. These primers can be synthesized by one of ordinary skill in the art, or can be purchased from commercial sources (see, for example, Stratagene, La Jolla, California), and these vendors sell murine and human variable region primers including V_{Ha}, V_{Hb}, V_{Hc}, V_{Hd}, C_{H1}, V_{L} and C_{L} region primers. These primers can be used to amplify heavy or light chain variable regions, which can then be inserted into vectors such as IMMUNOZAP^{™}H or ILLLFFLUNOZAP^{™}L (Stratagene). These vectors can then be introduced into E. coli, yeast, or mammalian-based expression systems. Large amounts of single-chain proteins comprising a fusion of V_{H} and V_{L} domains can be produced using these methods (see Bird et al., 1988, Science 242:423-426).

Once antibody-producing cells herein are obtained using any of the above-mentioned immunization and other techniques, specific antibody genes can be cloned by isolating the genes and amplifying DNA or mRNA therefrom according to standard methods described herein. The antibody produced therefrom is sequenced, and the CDRs are identified. The DNA encoding the CDRs can be manipulated as previously described to generate other antibodies provided herein.

The antibody provided herein preferably, in the cell-based assays described herein and/or in vivo assays described herein, modulates endothelin signaling and/or cross-block the binding of one of the antibodies described in the present application and/or be cross-blocked by binding to ET_{A}R via one of the antibodies described in the present application. Therefore, the assays described herein can be used to identify such binding agents.

In some embodiments, the antibody is generated by first identifying, in the cell-based and/or in vivo assays described herein, the antibody that binds to cells overexpressing ET_{A}R and/or neutralizes and/or cross-blocks the antibodies described in the present application and/or is cross-blocked by binding to ET_{A}R via one of the antibodies described in the present application.

It should be understood by one skilled in the art that certain proteins, such as antibodies, can undergo a variety of post-transcriptional modifications. The types and extents of these modifications depend on the host cell lines used to express the protein as well as the culture conditions. Such modifications include variations in glycosylation, methionine oxidation, diketopiperazine formation, aspartate isomerization and asparagine deamidation. Frequent modifications due to the action of carboxypeptidases lead to the loss of carboxy-terminal basic residues (such as lysine or arginine) (as described in Harris, 1995, Journal of Chromatography 705:129-134).

An alternative method of producing murine monoclonal antibodies is to inject hybridoma cells into the peritoneal cavity of syngeneic mice, for example, mice that have been treated (e.g., pristane primary immunization) to promote the formation of ascites containing monoclonal antibodies. Monoclonal antibodies can be isolated and purified by a variety of well-established techniques. Such isolation techniques include affinity chromatography with Protein A-sepharose, size exclusion chromatography, and ion exchange chromatography, see, e.g., Coligan, pages 2.7.1-2.7.12 and pages 2.9.1-2.9.3; Baines et al., "Purification of Immunoglobulin G (IgG)," Methods in Molecular Biology, vol. 10, pages 79-104 (The Humana Press, Inc., 1992). A monoclonal antibody can be purified by affinity chromatography using appropriate ligands screened on the basis of particular properties of the antibody (e.g., heavy or light chain isotype, binding specificity, etc.). Examples of appropriate ligands immobilized on a solid vector include Protein A, Protein G, an anti-constant region (light chain or heavy chain) antibody, an anti-idiotypic antibody, and a TGF-β binding protein, or a fragment or variant thereof.

Molecular evolution of the complementarity determining regions (CDRs) in the center of the antibody binding site has been used to isolate antibodies with increased affinities, for example, antibodies having increased affinities for c-erbB-2, as described by Schier et al., 1996, J. Mol. Biol. 263:551-567. Accordingly, such techniques are useful in preparing antibodies against human endothelin receptors.

Antibodies against human endothelin receptors can be used, for example, in assays to detect the presence or absence of the endothelin receptor, either in vitro or in vivo.

Antibodies can also be prepared by any of the conventional techniques. For example, these antibodies can be purified from cells that naturally express them (e.g., an antibody can be purified from a hybridoma that produces it) or produced in recombinant expression systems using any technique known in the art. See, for example, Monoclonal Antibodies, Hybridomas: A New Dimension in Biological Analyses, Kennet et al. (eds.), Plenum Press, (1980); and Antibodies: A Laboratory Manual, Harlow and Land (eds.), Cold Spring Harbor Laboratory Press (1988). This is discussed in the nucleic acid section below.

Antibodies can be prepared and screened for desired properties by any known techniques. Some techniques relate to the isolation of nucleic acids encoding polypeptide chains (or portions thereof) of related antibodies (e.g., anti-endothelin receptor antibodies) and the manipulation of the nucleic acids by recombinant DNA techniques. The nucleic acids can be fused with another relevant nucleic acid or modified (e.g., by mutagenesis or other conventional techniques) to add, delete or substitute one or more amino acid residues.

When it is desired to improve the affinity of antibodies comprising one or more of the above-mentioned CDRs herein, a variety of affinity maturation protocols can be used, including maintenance of CDRs (Yang et al., 1995, J. Mol. Biol. 254:392-403), chain shuffling (Marks et al., 1992, Bio/Technology 10:779-783), use of mutant strains of E. coli (Low et al., 1996, J. Mol. Biol. 250:350-368), DNA rearrangement (Patten et al., 1997, Curr. Opin. Biotechnol. 8:724-733), phage display (Thompson et al., 1996, J. Mol. Biol. 256:7-88) and additional PCR techniques (Crameri et al., 1998, Nature 391:288-291). All of these affinity maturation methods are discussed in Vaughan et al., 1998, Nature Biotechnology 16:535-539.

In one embodiment, the antibody provided herein is an anti-endothelin receptor fragment. The fragment may consist entirely of antibody-derived sequences or may comprise additional sequences. Examples of antigen binding fragments include Fab, F(ab')2, single-chain antibodies, diabodies, triabodies, tetrabodies and domain antibodies, and other examples are provided in Lunde et al., 2002, Biochem. Soc. Trans. 30:500-06.

Heavy and light chain variable domains (Fv regions) can be linked by an amino acid bridge (short peptide linker) to form a single-chain antibody, resulting in a single polypeptide chain. Such single-chain Fvs (scFvs) have been prepared by fusing DNA encoding a peptide linker between DNAs encoding the two variable domain polypeptides (V_{L} and V_{H}). The resulting polypeptides can fold back on themselves to form antigen binding monomers, or they can form polymers (e.g., dimers, trimers or tetramers), depending on the length of a flexible linker between the two variable domains (Kortt et al., 1997, Prot. Eng. 10:423; Kortt et al., 2001, Biomol. Eng. 18:95-108). By combining different V_{L} and V_{H} comprising polypeptides, multimeric scFvs that bind to different phenotypes can be formed (Kriangkum et al., 2001, Biomol. Eng. 18:31-40). Techniques developed for the production of single chain antibodies include those described in U.S. Pat. No. 4946778; Bird, 1988, Science 242:423; Huston et al., 1988, Proc. Natl. Acad. Sci. USA 85:5879-5883; Ward et al., 1989, Nature 334:544-546; de Graaf et al., 2002, Methods Mol Biol. 178:379-87. Single-chain antibodies derived from antibodies provided herein including, but not limited to, scFvs comprising the variable domain combination L1H1, are encompassed within the scopes described herein.

Antigen binding fragments derived from antibodies can also be obtained by proteolysis of the antibodies, for example, by pepsin or papain digestion of intact antibodies according to conventional methods. By way of example, antibody fragments can be produced by enzymatic cleavage of antibodies with pepsin to provide a SS fragment referred to as F(ab')2. This fragment can be further cleaved using a sulfhydryl reducing agent to produce 3.5S Fab' monovalent fragments. An alternative scheme is to perform the cleavage reaction with a sulfhydryl protecting group, resulting in the cleavage of the disulfide bond; in addition, enzymatic cleavage with papain directly produces two monovalent Fab fragments and an Fc fragment. These methods are described, for example, in Goldenberg, U.S. Pat. No. 4,331,647, Nisonoff et al., 1960, Arch. Biochem. Biophys. 89:230; Porter, 1959, Biochem. J. 73:119; Edelman et al., Methods in Enzymology l:422, (Academic Press, 1967); and Andrews and Titus, J.A. Current Protocols in Immunology (Coligan et al., (eds), John Wiley&Sons, 2003), pages 2.8,1-2.8.10 and pages 2.10A.1-2.10A.5. Other methods for cleaving antibodies, such as preparing heavy chains to form monovalent heavy and light chain fragments (Fds), further cleaving of fragments, or other enzymatic, chemical or genetic techniques can also be used, provided that the fragments bind to the antigen that is recognized by the intact antibody.

Another form of an antibody fragment is a peptide comprising one or more complementarity determining regions (CDRs) of an antibody. CDRs can be obtained by constructing polypeptides that encode the relevant CDRs. Such polypeptides can be prepared, for example, by using the polymerase chain reaction to synthesize the variable region using mRNA of antibody-producing cells as a template, see, for example, Larrick et al., 1991, Methods: A Companion to Methods in Enzymology 2:106; Courtenay-Luck, "Genetic Manipulation of Monoclonal Antibodies," Monoclonal Antibodies: Production, Engineering and Clinical Application, Ritter et al., (eds.), page 166 (Cambridge University Press, 1995); and Ward et al., "Genetic Manipulation and Expression of Antibodies," Monoclonal Antibodies: Principles and Applications, Birch et al., (eds.), page 137 (Wiley-Liss, Inc., 1995). The antibody fragment can further comprise at least one variable domain of the antibody described herein. Thus, for example, the V region domain can be monomeric and be a V_{H} or V_{L} domain, which can independently bind to an endothelin receptor with an affinity of at least 1 × 10⁻⁷ M or less as described below.

The variable region domain can be any natural variable domain or a genetically engineered version thereof. Genetically engineered version refers to a variable region domain produced by recombinant DNA engineering techniques. Such genetically engineered versions include those generated, for example, from a specific antibody variable region by insertions, deletions, or alterations in the amino acid sequences of the specific antibody. Particular examples include variable region domains comprising only one CDR and optionally one or more framework amino acids from one antibody and the remainder of the variable region domain from another antibody which are assembled by genetic engineering.

The variable region domain can be covalently linked at the C-terminal amino acid to at least one other antibody domain or a fragment thereof. Thus, by way of example, a V_{H} domain that is present in a variable region domain can be linked to an immunoglobulin CH1 domain or a fragment thereof. Similarly, a V_{L} domain can be linked to a C_{K} domain or a fragment thereof. In this way, for example, the antibody can be a Fab fragment, wherein the antigen binding domain comprises combined V_{H} and V_{L} domains covalently linked at the C-terminal thereof to CH1 and C_{K} domains, respectively. The CH1 domain can be extended with further amino acids, for example to provide a hinge region or a portion of a hinge region domain as found in a Fab' fragment, or to provide further domains, such as antibody CH2 and CH3 domains.

### Derivatives and variants of antibodies

The nucleotide sequences L1 and H1 encoding the amino acid sequence A-1 can be altered, for example, by random mutagenesis or by site-directed mutagenesis (e.g., oligonucleotide-induced site-directed mutagenesis) to generate an altered polynucleotide comprising one or more specific nucleotide substitutions, deletions, or insertions as compared to the non-mutated polynucleotide. Examples of techniques for making such alterations are described in Walder et al., 1986, Gene 42:133; Bauer et al., 1985, Gene 37:73; Craik, 1985, BioTechniques, 3:12-19; Smith et al., 1981, Genetic Engineering: Principles and Methods, Plenum Press; and U.S. Patent Nos. 4518584 and 4737462. These and other methods can be used to produce, for example, derivatives of anti-endothelin receptor antibodies with desired properties, such as enhanced affinity, avidity or specificity for endothelin receptors, enhanced in vivo or in vitro activity or stability, or reduced in vivo side effects as compared to the underivatized antibody.

Other derivatives of anti-endothelin receptor antibodies in the art include covalent or aggregated conjugates of anti-endothelin receptor antibodies or fragments thereof, with other proteins or polypeptides, for example, by expression of recombinant fusion proteins comprising heterologous polypeptides fused to the N-terminus or C-terminus of an anti-endothelin receptor antibody polypeptide. For example, the binding peptide can be a heterologous signal (or leader) polypeptide, e.g., a yeast α factor leader peptide or an epitope-tagged peptide. An antibody comprising fusion proteins can comprise peptides added to facilitate purification or identification of the antibody (e.g., polyhistidine). An antibody can also be linked to a FLAG peptide as described in Hopp et al., 1988, Bio/Technology 6:1204 and U.S. Pat. No. 5011912. The FLAG peptide is highly antigenic and provides an epitope reversibly bound by a specific monoclonal antibody (mAb), enabling rapid detection and facile purification of an expressed recombinant protein. Reagents useful for preparing fusion proteins in which the FLAG peptide is fused to a given polypeptide are commercially available (Sigma-Aldrich, St. Louis, MO). In another embodiment, oligomers that comprise one or more antibodies can be employed as endothelin receptor antagonists or higher oligomers. Oligomers can be in the form of covalently linked or non-covalently linked dimers, trimers, or higher oligomers. Oligomers comprising two or more antibodies may be used, with one example being a homodimer. Other oligomers include heterodimers, homotrimers, heterotrimers, homotetramers, heterotetramers, etc.

One embodiment is directed to oligomers comprising multiple antibodies, which are linked via covalent or non-covalent interactions between the peptide moieties fused to the antibodies. Such peptides may be peptide linkers (spacers), or peptides that have the property of promoting oligomerization. Leucine zippers and certain polypeptides derived from antibodies are peptides that can promote oligomerization of antibodies, as described in detail below.

In specific embodiments, oligomers comprise from two to four antibodies. The antibodies of the oligomers can be in any form, such as any of the forms described above, e.g., variants or fragments. Preferably, the oligomers comprise antibodies having endothelin receptor binding activity.

In one embodiment, oligomers are prepared using polypeptides derived from immunoglobulins. Preparation of fusion proteins comprising certain heterologous polypeptides fused to various portions of antibody-derived polypeptides (including the Fc domain) has been described, e.g., in Ashkenazi et al., 1991, PNAS USA 88:10535; Byrn et al., 1990, Nature 344:677; and Hollenbaugh et al., Construction of Immunoglobulin Fusion Proteins, Current Protocols in Immunology, Suppl. 4, pages 10.19.1-10.19.11. One embodiment herein is directed to a dimer comprising two fusion proteins produced by fusing an endothelin binding fragment of an anti-endothelin receptor antibody to the Fc region of the antibody. The dimer can be prepared in the following ways: for example, inserting a fusion gene encoding a fusion protein into an appropriate expression vector, expressing the fusion gene in host cells transformed with the recombinant expression vector, and allowing the expressed fusion protein to assemble like an antibody molecule, whereupon the inter-chain disulfide bonds between the Fc moieties form a dimer.

The term "Fc polypeptide" as used herein includes polypeptides in the form of natural proteins and mutant proteins derived from the Fc region of antibodies. Truncated forms of such polypeptides comprising the hinge region that promotes dimerization are also included. Fusion proteins comprising Fc moieties (and oligomers formed therefrom) offer the advantage of facile purification by affinity chromatography over Protein A or Protein G columns.

An appropriate Fc polypeptide, described in PCT application WO 93/10151 (incorporated herein by reference), is a single-chain polypeptide extending from the N-terminal hinge region to the natural C-terminus of the Fc region of a human IgG1 antibody. Another useful Fc polypeptide is the Fc mutant protein as described in U.S. Pat. No. 5457035 and in Baum et al., 1994, EMBO J. 13:3992-4001. The amino acid sequence of this mutant protein is identical to that of the natural Fc sequence as shown in WO 93/10151, except that amino acid 19 has been changed from leucine to alanine, amino acid 20 has been changed from leucine to glutamine, and amino acid 22 has been changed from glycine to alanine. The mutant protein exhibits reduced affinity for Fc receptors. In other embodiments, the heavy chain and/or light chain of an anti-endothelin receptor antibody may be substituted with the variable portion of the heavy chain and/or light chain thereof. Alternatively, the oligomer is a fusion protein comprising multiple antibodies, with or without peptide linkers (spacer peptides). These appropriate peptide linkers are described in U.S. Pat. No. 4751180 and 4935233.

Another method for preparing oligomeric antibodies involves use of a leucine zipper. Leucine zipper domains are peptides that promote oligomerization of the proteins in which they are present. Leucine zippers were originally found to be present in several DNA-binding proteins (Landschulz et al., 1988, Science 240:1759), and later found to be present in a variety of different proteins. Among the known leucine zippers are natural peptides or derivatives thereof that can be dimerized or trimerized. Examples of leucine zipper domains suitable for producing soluble oligomeric proteins are described in PCT application WO 94/10308, and the leucine zippers derived from pulmonary surfactant protein D (SPD) are described in Hoppe et al., 1994, FEBSLetters 344:191, incorporated herein by reference. The use of a modified leucine zipper that allows for stable trimerization of a heterologous protein fused thereto is described in Fanslow et al., 1994, Semin. Immunol. 6:267-78. In one method, recombinant fusion proteins comprising an anti-endothelin receptor antibody fragment or a derivative thereof fused to a leucine zipper peptide are expressed in appropriate host cells, and a soluble oligomeric anti-endothelin receptor antibody fragment or a derivative thereof is collected from the culture supernatant.

In another embodiment, the antibody derivative can comprise at least one of the CDRs disclosed herein. By way of example, one or more CDRs can be integrated into known antibody framework regions (IgG1, IgG2, etc.), or bind to appropriate vectors to enhance the half-life thereof. Appropriate vectors include, but are not limited to Fc, albumin, transferrin, etc. These and other appropriate vectors are known in the art. Such CDR conjugated peptides can be in monomeric, dimeric, tetrameric, or other forms. In one embodiment, one or more water-soluble polymers bind at one or more specific sites, for example at the amino terminus, of a binding agent. In an example, an antibody derivative comprises one or more water-soluble polymer attachments, including, but not limited to, polyethylene glycol, polyoxyethylene glycol, or polypropylene glycol. See, for example, U.S. Pat. Nos. 4640835, 4496689, 4301144, 4670417, 4791192 and 4179337. In some embodiments, the derivative comprises one or more of monomethoxy-polyethylene glycol, dextran, cellulose or other carbohydrate-based polymers, poly(N-vinyl pyrrolidone)-polyethylene glycol, polyoxyethylated polyols (e.g., glycerol) and polyvinyl alcohol, and mixtures of such polymers. In some embodiments, one or more water-soluble polymers are randomly attached to one or more side chains. In some embodiments, PEG can act to improve the therapeutic effect of a binding agent, such as an antibody. Certain such methods are described, for example, in U.S. Pat. No. 6133426, which is incorporated herein by reference for any purpose.

It should be understood that the antibody provided herein may have at least one amino acid substitution, provided that the antibody retains the binding specificity. Therefore, modifications of antibody structures are encompassed within the scopes described herein. These modifications can include amino acid substitutions, which may be conservative or non-conservative, that do not destroy the endothelin receptor binding ability of an antibody. Conservative amino acid substitutions may include non-natural amino acid residues, which are generally integrated by chemical peptide synthesis rather than by synthesis in biological systems. These amino acid residues include peptidomimetics and other reversed or inverted forms of amino acid moieties. Conservative amino acid substitutions can also involve a substitution of a natural amino acid residue with a non-natural residue such that there is little or no effect on the polarity or charge of the amino acid residue at that site. Non-conservative substitutions can involve the exchange of a member of one class of amino acids or amino acid analogs for a member from another class with different physical properties (e.g., size, polarity, hydrophobicity and charge).

Moreover, one skilled in the art may generate variants to be tested, which contain an amino acid substitution at each desired amino acid residue. The variants can be screened using activity assays known to one skilled in the art. Such variants can be used to gather information about appropriate variants. By way of example, if it is found that a change in a certain amino acid residue results in destroyed, undesirably reduced, or unsuitable activity, variants with such a change may be avoided. In other words, based on information gathered from such routine experiments, one skilled in the art can readily determine the amino acids where further substitutions should be avoided either alone or in combination with other mutations.

One skilled in the art will be able to determine appropriate variants of the polypeptide as listed herein using known techniques. In some embodiments, one skilled in the art may identify appropriate regions of the molecule that may be altered without destroying the activity by targeting regions not to be important for activity. In some embodiments, residues or portions of molecules that are conserved among similar polypeptides can be identified. In some embodiments, even conservative substitutions for regions that are important for biological activity or structure do not destroy the biological activity or have an adverse effect on the polypeptide structure. Additionally, one skilled in the art can review structure- function studies to identify residues in similar polypeptides that are important for activity or structure. In view of such a comparison, the importance of amino acid residues in a protein that correspond to amino acid residues which are important for activity or structure in similar proteins can be predicted. One skilled in the art may choose chemically similar amino acid substitutions for such predicted important amino acid residues.

One skilled in the art can also analyze the three-dimensional structure and amino acid sequence in relation to that structure in similar polypeptides. In view of such information, one skilled in the art may predict the alignment of amino acid residues of an antibody with respect to the three-dimensional structure thereof. In some embodiments, one skilled in the art may choose not to make significant alteration to amino acid residues predicted to be on the surface of a protein, because such residues may be involved in important interactions with other molecules. A number of scientific publications have been devoted to the prediction of secondary structure. See, Moult, 1996, Curr. Op. Biotech. 7:422-427, Chou et al., 1974, Biochemistry 13:222-245; Chou et al., 1974, Biochemistry 113:211-222; Chou et al., 1978, Adv. Enzymol. Relat. Areas Mol. Biol. 47:45-148; Chou et al., 1979, Ann. Rev. Biochem. 47:251-276, and Chou et al., Biophys. J. 26:367-384. Moreover, computer programs are currently available to assist with predicting secondary structure. By way of example, two polypeptides or proteins which have a sequence identity greater than 30%, or similarity greater than 40% often have similar high-level structures. The recent growth of the Protein Data Bank (PDB) has provided enhanced predictability of secondary structure, including the potential number of folds within the structure of a polypeptide or protein. See, Holm et al., 1999, Nucl. Acid. Res. 27:244-247. It has been suggested (Brenner et al., 1997, Curr. Op. Struct. Biol. 7:369-376) that there are a limited number of folds in a given polypeptide or protein and that once a critical number of structures are determined, the structural prediction will become dramatically more accurate.

Additional methods for predicting secondary structure include "threading" (Jones, 1997, Curr. Opin. Struct. Biol., 7:377-87; Sippl et al., 1996, Structure 4:15-19) "profile analysis" (Bowie et al., 1991, Science 253:164-170; Gribskov et al., 1990, Meth. Enzym. 183:146-159; Gribskov et al., 1987, Proc. Nat. Acad. Sci. USA 84:4355-4358), and "evolutionary linkage" (see Holm, supra (1999), and Brenner, supra (1997)). In some embodiments, antibody variants include glycosylation variants, wherein the number and/or type of glycosylation sites have been altered compared to the amino acid sequences of a parent polypeptide. In some embodiments, variants comprise a greater or lesser number of N-linked glycosylation sites than natural proteins. Alternatively, a substitution that removes this sequence may remove an existing N-linked carbohydrate chain. Also provided is a rearrangement of N-linked carbohydrate chains, wherein one or more N-linked carbohydrate chain sites (generally those that are naturally occurring) are removed and one or more new N-linked sites are created. Additional preferred antibody variants include cysteine variants, wherein one or more cysteine residues are deleted or substituted with another amino acid (e.g., serine) as compared to the parent amino acid sequence. Cysteine variants can be useful when antibodies must be folded into a biologically active conformation (for example, after isolation of soluble inclusion bodies). Cysteine variants generally have fewer cysteine residues than natural proteins, and generally have an even number of cysteines to minimize interactions resulting from unpaired cysteines.

Desired amino acid substitutions (whether conservative or non-conservative) can be determined by one skilled in the art at the time such substitutions are desired. In some embodiments, amino acid substitutions can be used to identify important residues of human endothelin receptor antibodies, or to increase or decrease the affinity of the human endothelin receptor antibodies described herein.

According to some embodiments, preferred amino acid substitutions are those which: (1) reduce susceptibility to proteolysis, (2) reduce susceptibility to oxidation, (3) alter the binding affinity for forming protein complexes, (4) alter the binding affinity, and/or (4) confer or modify other physiochemical or functional properties on such polypeptides. According to some embodiments, single or multiple amino acid substitutions (in some embodiments, conservative amino acid substitutions) may be performed in naturally occurring sequences (in some embodiments, in the portion of the polypeptide outside the domains that form intermolecular contacts). In some embodiments, conservative amino acid substitutions generally cannot substantially alter the structural characteristics of a parent sequence (e.g., the substitution of amino acids shall not destroy a helix present in the parent sequence or interfere with other types of secondary structure that characterize the parent sequence). Examples of art-recognized polypeptide secondary and tertiary structures are described in Proteins, Structures and Molecular Principles, Creighton (eds.), W.H. Freeman and Company (1984); Introduction to Protein Structure, Branden and Tooze (eds.), Garland Publishing (1991); and Thornton et al., 1991, Nature 354:105, each of which is incorporated herein by reference.

In some embodiments, the antibody provided herein can be chemically bonded with polymers, lipids, or other moieties.

An antigen binding agent may comprise at least one of the CDRs described herein incorporated into a biocompatible framework structure. In an example, the biocompatible framework structure comprises a polypeptide or a portion thereof that is sufficient to form a conformationally stable structural support, or framework, or scaffold, which is able to present one or more amino acid sequences (e.g., CDRs, variable regions, etc.) that bind to an antigen in a localized surface region. Such structures can be naturally occurring polypeptides or polypeptide "folds" (structural motifs), or can have one or more modifications, such as additions, deletions or substitutions of amino acids, relative to a naturally occurring polypeptide or fold. These scaffolds can be derived from a polypeptide of any species (or of more than one species), such as humans, other mammals, other vertebrates, invertebrates, bacteria or viruses.

Generally, biocompatible framework structures are based on protein scaffolds or frameworks rather than immunoglobulin domains. By way of example, those protein scaffolds based on fibronectin, ankyrin, lipocalin, neocarzinostatin, cytochrome b, CP1 zinc finger protein, PST1, coiled-coil protein, LACI-D1, Z domain and tendamistat domain can be used (see, for example, Nygren and Uhlen, 1997, Current Opinion in Structural Biology 7:463-469).

Additionally, one skilled in the art will recognize that appropriate binding agents include portions of these antibodies, such as one or more of the heavy chain CDR1s, CDR2s and CDR3s, and light chain CDR1s, CDR2s and CDR3s as specifically disclosed herein. At least one of the heavy chain CDR1, CDR2, CDR3, light chain CDR1, CDR2 and CDR3 regions has at least one amino acid substitution, provided that the antibody retains the binding specificity of the non-substituted CDR. The non-CDR moiety of the antibody may be a non-protein molecule, wherein the binding agent cross-blocks the binding of the antibody disclosed herein to human ET_{A}R and/or inhibits endothelin signaling via the receptor. The non-CDR moiety of the antibody may be a non-protein molecule, wherein the antibody exhibits a binding type similar to that of at least one of the antibodies A-1/A-2 to human ET_{A}R peptide, and/or neutralizes the activity of endothelin in a competitive binding assay. The non-CDR moiety of an antibody may be composed of amino acids, wherein the antibody is a recombinant binding protein or a synthetic peptide, and the recombinant binding protein cross-blocks the binding of the antibody disclosed herein to human ET_{A}R and/or neutralizes the activity of endothelin in vivo or in vitro. The non-CDR moiety of an antibody may be composed of amino acids, wherein the antibody is a recombinant antibody, and the recombinant antibody exhibits a binding type similar to that of at least one of the antibodies A-1/A-2 to human ET_{A}R peptide, and/or neutralizes endothelin signaling in a competitive binding assay.

### Nucleic acids

In one aspect, provided herein is an isolated nucleic acid molecule. The nucleic acid molecule comprises, for example, polynucleotides that encode all or part of an antibody, such as one or both chains of the antibody described herein, or a fragment, derivative, mutant protein or variant thereof; polynucleotides sufficient for use as hybridization probes; PCR primers or sequencing primers for identifying, analyzing, mutating or amplifying a polynucleotide encoding a polypeptide; anti-sense nucleic acids for inhibiting expression of a polynucleotide, and complementary sequences thereof. The nucleic acid may be in any length. The nucleic acid may be, for example, 5, 10, 15, 20, 25, 30, 35, 40, 45, 50, 75, 100, 125, 150, 175, 200, 250, 300, 350, 400, 450, 500, 750, 1000, 1500, 3000, 5000 or more nucleotides in length, and/or comprise one or more additional sequences, for example, regulatory sequences, and/or be one portion of a larger nucleic acid, for example, a vector. The nucleic acid may be single-stranded or double-stranded and comprise RNA and/or DNA nucleotides, and artificial variants thereof (e.g., peptide nucleic acids).

Nucleic acids encoding antibody polypeptides (e.g., heavy or light chain, variable domain only, or full length) can be isolated from B cells of mice that have been immunized with an ET_{A}R antigen. The nucleic acid can be isolated by conventional methods such as polymerase chain reaction (PCR).

The nucleic acid sequences encoding the heavy and light chain variable regions are shown above. The skilled artisan will appreciate that, due to the degeneracy of the genetic code, each of the polypeptide sequences disclosed herein can be encoded by a large number of other nucleic acid sequences. Provided herein is each degenerate nucleotide sequence encoding the antibody provided herein.

Further provided herein are nucleic acids that hybridize to other nucleic acids (e.g., nucleic acids comprising a nucleotide sequence of any of A-1/A-2) under specific hybridization conditions. Methods for hybridizing nucleic acids are well-known in the art. See, for example, Current Protocols in Molecular Biology, John Wiley & Son (1989), 6.3.1-6.3.6. As defined herein, for example, moderately stringent conditions use a prewashing solution containing 5×sodium chloride/sodium citrate (SSC), 0.5% SDS, 1.0 mM EDTA (pH 8.0), a hybridization buffer of approximately 50% formamide, 6×SSC, a hybridization temperature of 55° C (or other similar hybridization solutions, such as a solution containing approximately 50% formamide, with a hybridization temperature of 42°C), and elution conditions of 60°C, 0.5×SSC and 0.1% SDS. Stringent hybridization conditions use hybridization in 6×SSC at 45°C, followed by one or more washes in 0.1×SSC and 0.2% SDS at 68°C. Furthermore, one skilled in the art can manipulate hybridization and/or washing conditions to increase or decrease the stringency of hybridization such that nucleic acids comprising nucleotide sequences that are at least 65%, 70%, 75%, 80%, 85%, 90%, 95%, 98% or 99% homologous to each other generally remain hybridized to each other. The basic parameters affecting the choice of hybridization conditions and guidance for designing appropriate conditions are listed in, for example, Sambrook, Fritsch and Maniatis, 1989, Molecular Cloning: A Laboratory Manual, Cold Spring Harbor Laboratory Press, chapters 9 and 11, and Current Protocols in Molecular Biology, 1995, Ausubel et al., (eds), John Wiley & Sons, Inc., sections 2.10 and 6.3-6.4, and can be readily determined by one of ordinary skill in the art on the basis of, for example, the length and/or base composition of DNA. Changes can be introduced by means of mutations into a nucleic acid, thereby leading to changes in the amino acid sequence of the polypeptide (e.g., an antigen binding protein) encoded thereby. Mutations can be introduced using any technique known in the art. In one embodiment, one or more specific amino acid residues are altered using, for example, a site-directed mutagenesis protocol. In another embodiment, one or more randomly selected residues are altered using, for example, a random mutagenesis protocol. No matter how it is made, a mutant polypeptide can be expressed and screened for desired properties.

Mutations can be introduced into a nucleic acid without significantly altering the biological activity of a polypeptide encoded thereby. For example, nucleotide substitutions leading to amino acid substitutions at non-essential amino acid residues can be made. In one embodiment, nucleotide sequences provided herein for L-1 to L-2 and H-1 to H-2, or fragments, variants, or derivatives thereof, are mutated such that the amino acid sequence encoded thereby comprises, as compared to that encoded by L-1 to L-2 and H-1 to H-2 as shown herein, one or more deletions or substitutions of amino acid residues, resulting in sequences involving two or more different residues. In another embodiment, mutagenesis leads to insertion of an amino acid adjacent to one or more amino acid residues of L-1 to L-2 and H-1 to H-2 as shown herein, resulting in sequences involving two or more different residues. Alternatively, one or more mutations can be introduced into a nucleic acid to selectively alter the biological activity (e.g., binding to ET_{A}R) of a polypeptide encoded thereby. For example, the mutation can quantitatively or qualitatively change the biological activity. Examples of quantitative changes include increasing, reducing or eliminating the activity. Examples of qualitative changes include changing the antigen specificity of the antibody.

In another aspect, provided herein are nucleic acid molecules that are suitable for use as primers or hybridization probes for the detection of nucleic acid sequences herein. The nucleic acid molecule herein can comprise only a portion of a nucleic acid sequence encoding the full-length polypeptide herein, for example, a fragment that can be used as a probe or a primer or a fragment encoding an active moiety (e.g., ET_{A}R binding moiety) of the polypeptide herein.

Probes based on the nucleic acid sequence herein can be used to detect the nucleic acid or similar nucleic acids, for example, transcripts encoding the polypeptide herein. The probes may comprise a labelling group, e.g., a radioisotope, a fluorescent compound, an enzyme, or an enzyme cofactor. Such probes can be used to identify a cell that expresses the polypeptide.

In another aspect, provided herein is a vector comprising a nucleic acid encoding the polypeptide herein or a portion thereof. Examples of vectors include, but are not limited to, plasmids, viral vectors, non-episomal mammalian vectors and expression vectors, for example, recombinant expression vectors.

The recombinant expression vectors herein can comprise the nucleic acid herein in a form suitable for expression of the nucleic acid in a host cell. The recombinant expression vector includes one or more regulatory sequences, which are screened on the basis of the host cells used for expression, and are operably linked to the pre-expressed nucleic acid sequence. Regulatory sequences include those that direct the constitutive expression of nucleotide sequences in many types of host cells (e.g., an SV40 early gene enhancer, Rous sarcoma virus promoter and cytomegalovirus promoter), those that direct the expression of nucleotide sequences only in certain host cells (e.g., a tissue-specific regulatory sequence, see Voss et al., 1986, Trends Biochem. Sci. 11:287, Maniatis et al., 1987, Science 236:1237, the disclosure of each of which is incorporated by reference herein in its entirety), and those that direct the inducible expression of nucleotide sequences in response to specific treatments or conditions (e.g., a metallothionin promoter in mammalian cells and a tet-responsive promoter and/or streptomycin responsive promoter in both prokaryotic and eukaryotic systems (supra)). It should be understood by one skilled in the art that the design of the expression vector can depend on factors such as the choice of the host cell to be transformed, and the level of expression of the protein desired. The expression vector herein can be introduced into a host cell, thereby producing the protein or peptide, including a fusion protein or peptide, encoded by the nucleic acid described herein.

In another aspect, provided herein is a host cell into which the expression vector herein can be introduced. The host cell may be any prokaryotic or eukaryotic cell. Prokaryotic host cells include Gram-negative or Gram-positive organisms, for example, E. coli or bacilli. Higher eukaryotic cells include insect cells, yeast cells, and established cell lines of mammalian origin. Examples of appropriate mammalian host cell lines include Chinese hamster ovary (CHO) cells or derivatives thereof such as Veggie CHO and related cell lines which grow in serum-free media (see Rasmussen et al., 1998, Cytotechnology 28:31) or CHO strain DXB-11 which is deficient in DHFR (see Urlaub et al., 1980, Proc. Natl. Acad, Sci. USA 77:4216-20). Additional CHO cell lines include CHO-K1 (ATCC #CCL-61), EM9 (ATCC #CRL-1861) and UV20 (ATCC #CRL-1862). Additional host cells include monkey kidney COS-7 cell lines (ATCC #CRL-1651) (see Gluzman et al., 1981, Cell 23:175), L cells, C127 cells, 3T3 cells (ATCC CCL-163), AM-1/D cells (described in U.S. Patent serial No. 6210924), HeLa cells, BHK (ATCC CRL-10) cell lines, the CV1/EBNA cell line derived from the African green monkey kidney cell line CV1 (ATCC CCL-70) (see McMahan et al., 1991, EMBO J. 10:2821), human embryonic kidney cells such as 293, 293 EBNA or MSR 293, human epidermal A431 cells, human C010205 cells, other transformed primate cell lines, normal diploid cells, cell strains derived from in vitro culture of primary tissue, primary explants, HL-60, U937, HaK or Jurkat cells. Appropriate cloning and expression vectors for bacterial, fungal, yeast, and mammalian cell hosts are described in Pouwels et al. (Cloning Vectors: A Laboratory Manual, Elsevier, 1985).

Vector DNA can be introduced into prokaryotic or eukaryotic cells by conventional transformation or transfection techniques. For stable transfection of mammalian cells, it is known that, depending upon the expression vector and transfection technique used, only a small fraction of cells can integrate the foreign DNA into the genome thereof. In order to identify and screen for these integrants, a gene that encodes a selectable marker (e.g., antibiotic resistance) is generally introduced into a host cell along with the gene of interest. Preferred selectable markers include those which confer resistance to drugs, such as G418, hygromycin and methotrexate. Among other methods, cells stably transfected with the introduced nucleic acid can be identified by drug screening (for example, cells that have integrated the selectable gene will survive, while other cells die).

The transformed cells can be cultured under conditions that promote expression of a polypeptide, and the polypeptide can be recovered by conventional protein purification methods. One such purification method is described in the examples below. Polypeptides contemplated for use herein include substantially homogeneous recombinant mammalian anti-endothelin receptor antibody polypeptides, which are substantially free of contaminating endogenous materials.

### Antibody activity

In one embodiment, the antibody provided herein specifically binds to endothelin receptors, inhibiting signaling. In another embodiment, provided herein is a murine antibody or a humanized antibody capable of specifically binding to a human endothelin receptor. Such antibodies include antagonistic or neutralizing antibodies that can reduce or neutralize endothelin signaling.

In one embodiment, the antibody provided herein binds to human endothelin receptor ET_{A}R with a K_{d} of approximately 0.01 nM to 1000 nM, 0.1 nM to 500 nM, 0.5 nM to 200 nM, 1 nM to 200 nM, or 10 nM to 100 nM. In another embodiment, the antibody provided herein binds to human endothelin receptor ET_{A}R with a K_{d} of approximately 1 nM to 200 nM. In another embodiment, the antibody provided herein binds to human endothelin receptor ET_{A}R with a K_{d} of approximately 10 nM to 100 nM. In another embodiment, the antibody provided herein binds to human endothelin receptor ET_{A}R with a K_{d} of approximately 1 nM, 2 nM, 5 nM, 10 nM, 20 nM, 30 nM, 40 nM, 50 nM, 60 nM, 70 nM, 80 nM, 90 nM or 100 nM.

In one embodiment, the antibody provided herein has an IC₅₀ value of approximately 0.01 nM to 500 nM, 0.1 nM to 200 nM, 0.5 nM to 200 nM, 1 nM to 200 nM, or 10 nM to 100 nM in reducing human endothelin signaling. In another embodiment, the antibody provided herein has an IC₅₀ value of approximately 1 nM to 200 nM in reducing human endothelin signaling. In another embodiment, the antibody provided herein has an IC₅₀ value of approximately 10 nM to 100 nM in reducing human endothelin signaling. In another embodiment, the antibody provided herein has an IC₅₀ value of approximately 1 nM, 2 nM, 5 nM, 10 nM, 20 nM, 30 nM, 40 nM, 50 nM, 60 nM, 70 nM, 80 nM, 90 nM or 100 nM.

In one embodiment, the ET_{A}R antibody provided herein, when binding to human endothelin receptor ET_{A}R, has one or more of the following properties:
a. providing the same K_{d} as that of the reference antibody when binding to human endothelin receptor ET_{A}R;
b. providing the same IC₅₀ as that of the reference antibody when inhibiting the activation of human endothelin receptor ET_{A}R; and
c. cross-competing with the reference antibody for binding to human endothelin receptor ET_{A}R.

In one aspect, the reference antibody comprises a combination of the light chain variable domain amino acid sequence SEQ ID NO: 138 and the heavy chain variable domain amino acid sequence SEQ ID NO: 166. In another aspect, the reference antibody is a monoclonal antibody A-1, A-2, A-7, A-9 or A-12.

Herein, the term "substantially similar" means that the IC₅₀ or K_{d} is comparable to that of the reference antibody, or the IC₅₀ or K_{d} value is 200%, 180%, 160%, 150%, 140%, 120%, 110%, 100%, 99%, 98%, 97%, 95%, 90%, 85%, 80%, 75%, 70%, 65% or 50% of that of the reference antibody. In one embodiment, the reference antibody includes, for example, an antibody having a heavy chain and light chain combination L1H1 or L2H2. In one embodiment, the reference antibody includes an ET_{A}R antibody A-1.

### Indication

In one embodiment, the change in the biological activity of an ET_{A}R antibody is detected by calcium flux experiment to test the function of the ET_{A}R antibody to inhibit ET_{A}R in vitro.

In one embodiment, the in vivo biological activity of an ET_{A}R antibody is detected by using acute renal injury models and diabetic nephropathy animal models to examine the therapeutic effect of the ET_{A}R antibody on kidney diseases.

The present invention provides an antibody capable of specifically binding to human endothelin receptor and blocking ET-1 and ET_{A}R signaling, so as to treat diabetic nephropathy, chronic nephropathy, a disease accompanied by proteinuria or a disease accompanied by a decrease in glomerular filtration rate.

### Treatment method

Herein, the term "subject" refers to mammals, including humans, and is used interchangeably with the term "patient". The term "treatment" includes alleviating or preventing at least one symptom or other aspects of a condition, or alleviating the severity of a disease, etc. The antibody provided herein can constitute an effective therapeutic agent without the need to produce a complete cure effect or eradicate all symptoms or manifestations of a disease. As recognized in the related art, a drug as a therapeutic agent can be used to reduce the severity of a given disease state, but does not need to eliminate all manifestations of the disease to be considered as an effective therapeutic agent. Similarly, prophylactic administration treatment does not need to be completely effective in preventing the appearance of symptoms to constitute an effective prophylactic agent. It is sufficient to reduce the impact of a disease (e.g., by reducing the number or severity of symptoms thereof, or by enhancing another therapeutic effect, or by producing another effective effect), or to reduce the likelihood of occurrence or aggravation of the disease in a subject. One embodiment herein relates to a method comprising administering an antibody to a patient in an amount and time sufficient to induce a sustained improvement of an indicator that reflects the severity of a specific condition above the baseline level.

Pharmaceutical compositions may be administered by any appropriate technique including but not limited to parenteral, topical or inhalation administration. In the case of injection, a pharmaceutical composition can be administered by, for example, intra-articular, intravenous, intramuscular, intralesional, intraperitoneal or subcutaneous route, as a rapid injection or continuous infusion. For example, local administration at the site of disease or injury may be considered, such as transdermal administration and sustained release administration of implants. Inhalation administration includes, for example, nasal or oral inhalation, sprays, inhalation of antibodies in the form of aerosols, etc. Other options include oral preparations including tablets, syrups or lozenges.

It is advantageous to administer the antibody provided herein in the form of a composition comprising one or more other components, such as a physiologically acceptable carrier, excipient or diluent. The composition may optionally comprise one or more additional physiologically active agents as described below. In various specific embodiments, the composition comprises one, two, three, four, five or six physiologically active agents other than one or more antibodies (such as a murine antibody or a humanized antibody) provided herein.

The dosage and frequency of administration can vary based on the following factors: the route of administration, the specific antibody used, the nature and severity of the disease to be treated, whether the symptoms are acute or chronic, and the size and overall symptoms of the patient. The appropriate dose can be determined by methods well known in the art, for example, dose scaling studies in clinical trials.

The antibody provided herein can be administered, for example, once or several times at regular intervals over a period of time. In specific embodiments, a murine antibody or a humanized antibody is administered once in at least one month or longer, for example, one, two, or three months or even indefinitely. For the treatment of chronic symptoms, long-term treatment is usually the most effective. However, for the treatment of acute symptoms, short-term administration, for example, from one week to six weeks is sufficient. Generally, human antibodies are administered until a patient shows a medically relevant improvement of the selected sign or an indicator above the baseline level.

Any change in the symptoms of a patient can be monitored before, during and/or after treatment with an antibody, e.g., a human antibody. For some conditions, proteinuria and changes in glomerular filtration rate can vary with factors such as disease progression.

Specific embodiments of the methods and compositions provided herein involve the use of, for example, an antibody and one or more endothelin antagonists, two or more antibody provided herein, or the antibody of the present invention and one or more other endothelin antagonists. In a further embodiment, an antibody is administered alone or in combination with other agents for treating distressing symptoms of the patient. Examples of these agents include proteins and non-protein drugs. When multiple drugs are administered in combination, the dosage thereof should be adjusted accordingly as is well known in the art. "Combined administration" combination therapy is not limited to simultaneous administration, and also includes a treatment regimen in which an antigen and a protein are administered at least once during a course of treatment involving the administration of at least one other therapeutic agent to a patient.

Provided herein are compositions, kits and methods related to an antibody capable of specifically binding to a human endothelin receptor. Also provided are nucleic acid molecules, and derivatives and fragments thereof, comprising a polynucleotide encoding all or a portion of a polypeptide that binds to an endothelin receptor, such as a nucleic acid encoding all or a portion of an anti-endothelin receptor antibody, antibody fragment, or antibody derivative. Further provided herein are vectors and plasmids comprising such nucleic acids, and cells and cell lines comprising such nucleic acids and/or the vectors and plasmids. The methods provided include, for example, methods for preparing, identifying or isolating an antibody, e.g., an anti-ET_{A}R antibody, that binds to human ET_{A}R, and methods for determining whether the antibody binds to ET_{A}R.

The following specific examples are used to further illustrate the technical solutions herein.

### Detailed Description of Embodiments:

Herein, unless otherwise specified, the raw materials, equipment and the like used can all be purchased from the market or are commonly used in the art. The methods of the following examples, if not indicated specifically, are all conventional methods in the art.

### 1. Construction of stable antigen cell line for immunization

CHO-DHFR- cells were seeded into a 6-well plate, cultured for 24 h, and then transfected with pIRES plasmid (Clontech) cloned with hET_{A}R gene (see SEQ ID NO: 1 for the nucleotide sequence, and see SEQ ID NO: 2 for the amino acid sequence), wherein transfection was performed according to the transfection conditions recommended by Invitrogen for Lipofectamine 2000. After 48 h, the culture medium was changed to a complete culture medium containing 10 nM MTX (methotrexate) every 3 days. After about two weeks of culture, stable clones appeared. The cell colonies were digested and dispersed. The cells were passaged, and continued to be cultured. When the passage cells grew to 50% confluence, pressure screening was performed respectively by gradually increasing the concentration of MTX until the concentration of MTX was 10 µM. The constructed stable cell strains were separately detected by FACS using polyclonal ET_{A}R antibody (Abcam), and the cell populations after pressure screening were identified according to the FACS detection results. After screening, a large amount of hET_{A}Rs were expressed on CHO-DHFR-hET_{A}R cell membranes. Finally, after subcloning and further identification, 6 strains of ET_{A}R cells were highly-expressed stable cell lines.

### 2. Preparation of antibody

CHO-DHFR-hET_{A}R whole cells emulsified in Freund's adjuvant were injected subcutaneously into BALB/c mice (6-8 weeks old) at a dose of 2 × 10⁶ cells/mouse. After 2 weeks, the mice were boosted with incomplete Freund's adjuvant emulsified immunogen, and then boosted once a week. After a total of 6 immunizations, blood was collected by tail snipping. Serum was isolated by centrifugation and serum titers were determined by FACS. When appropriate antibody titers were reached, mice were sacrificed by cervical dislocation and spleen cells were obtained aseptically. SP2/0 cells in logarithmic phase were collected, and centrifuged at 2000 rpm for 3 min. The cell precipitate was resuspended in a serum-free culture medium, then centrifuged and resuspended for a second time, and counted. Spleen cells and SP2/0 cells were mixed, ensuring SP2/0 cells : spleen cells ≥ 1 : 1. After mixing, 3 rounds of washing-centrifugation were performed. After the cell precipitate from the last centrifugation was detached, 1 mL of pre-warmed PEG-1350 was added dropwise (finished in 30 s). Pipette-mixing was performed for 1 min, and then 30 mL of a pre-warmed serum-free culture medium was added slowly to terminate the PEG fusion. After centrifugation for 5 min at 1500 rpm, the cell precipitate was detached, and added with a fusion culture medium. 20000 spleen cells and 5000 feeder layer cells were plated into a 96-well plate per well, with 100 µL of culture medium per well. The fused hybridoma cells and feeder layer cells were co-cultured in the 96-well plate, and subjected to HAT (hypoxanthine, methotrexate and thymidine) screening to remove non-fused cells. After 10 days, the hybridoma cell supernatants in the culture plate were harvested for ELISA detection.

### 3. ELISA screening of whole cells

CHO-DHFR-hET_{A}R cells over-expressing hET_{A}R and CHO-DHFR- cells not expressing hET_{A}R were seeded separately into 96-well plates. When the cells grew to 90% confluence, the cell culture supernatant was removed, and the cells were washed twice with PBS, added with 100 µL of 100% methanol, and fixed for 10 min at 4°C. Then 100 µL of freshly made 0.6% H₂O₂-PBS was added for treatment for 20 min at room temperature, and the cells were washed twice with PBS. After blocking with PBS-1% BSA, the cells were added with the hybridoma cell supernatant and incubated for 90 min at 4°C. After several washes, 100 µL of diluted GxM-HRP-Fc secondary antibody (Sigma-Aldrich) was added to each well, and the cells were incubated at 37°C for 0.5 hr. After 5 washes, 100 µL of a TMB chromogenic substrate was added to each well, reaction was performed at 37°C for 15 min, 2M H₂SO₄ was added for terminating colour development, and OD₄₅₀ values were read. The positive control was the serum of immunized mice. The negative control was the cell culture medium supernatant. After initial detection by ELISA, several positive hybridoma cell strains secreting anti-hET_{A}R antibody were screened. These hybridoma strains secreting anti-hET_{A}R antibody were selected and cloned to obtain cell strains capable of stably secreting anti-hET_{A}R antibody. Finally, the supernatant of the antibody secreted by the hybridoma cells was selected for verification by FACS.

### 4. Cloning and subcloning of antibody gene

Hybridoma cells secreting antibodies were collected, and according to the manufacturer protocol of QIAGEN mRNA extraction kit, the mRNA of the hybridoma cells was extracted. Then the extracted mRNA was transcribed reversely into cDNA. The reverse transcription primers were specific primers for the light and heavy chain constant regions of a mouse, with the heavy chain reverse transcription primer being (5'-TTTGGRGGGAAGATGAAGAC-3') (SEQ ID NO: 199), and the light chain reverse transcription primers being (5'-TTAACACTCTCCCCTGTTGA A-3') (SEQ ID NO: 200) and (5'-TTAACACTCATTCCTGTTGAA-3') (SEQ ID NO: 201). The reaction conditions of RT-PCR were: 25°C 5 min; 50°C 60 min; 70°C 15 min. The reversely-transcribed cDNA was diluted to 500 µL with 0.1 mM TE, added to an ultrafiltration centrifuge tube (Amicon Ultra-0.5), and centrifuged at 2000 g for 10 min; the filtrate was discarded, and 500 µL of 0.1 mM TE was added and centrifuged at 2000 g for 10 min; the filtrate was discarded, and the preparation tube was inverted into a new centrifuge tube and centrifuged at 2000 g for 10 min to obtain purified cDNA; 10 µL of the purified cDNA was taken as a template, and 4 µL of 5×tailing buffer (Promega), 4 µL of dATP (1 mM) and 10U terminal transferase (Promega, commercially available) were added, mixed uniformly, and incubated at 37°C for 5 min and then incubated at 65°C for 5 min; and then the cDNA with PolyA tail was used as a template to amplify the genes of the light and heavy chain variable regions of the antibody by PCR. The upstream primers were all OligodT, and the downstream primers of the heavy chain were (5'-TGGACAGGGATCCAGAGTTCC-3') (SEQ ID NO: 202) and (5'-TGGACAGGGCTCCATAGTTCC-3') (SEQ ID NO: 203), and the downstream primer of the light chain was (5'-ACTCGTCCTTGGTCAACGTG-3') (SEQ ID NO: 204). The reaction conditions of PCR were: 95°C 5 min; 95°C 30 s, 56°C 30 s, 72°C 1 min 40 cycles; 72°C 7 min. The PCR product was linked to PMD 18-T vector (Takara Bio) and then sequenced. The sequences of the cloned antibody were as shown in Table 2.

PCR primers were designed on the basis of the sequenced DNA sequences of the antibody, and thus the complete light chain and heavy chain signal peptides and variable domains and mouse IgG1 constant region were linked to expression vector pTM5.

### 5. Antibody humanization and optimization

First of all, the screened murine antibody light and heavy chain variable region sequences were obtained, humanized antibody germline gene sequences (Ig Germline Gene sequence) homologous to the entered antibody variable region sequences were searched using NCBI online antibody variable region sequence alignment tool (Ig Blast), and the humanized gene sequences having the highest homology except CDR sequences were used as templates for CDR grafting to obtain humanized antibody variable region sequences. The genes for the humanized antibody light and heavy chains were synthesized by outsourcing. According to the sequence, PCR primers were designed, appropriate restriction enzyme sites were introduced at the 5' end and 3' end of the synthetic sequence, and after amplification by PCR, the sequence was spliced to a human IgG2 or IgG4 constant region sequence to obtain the complete recombinant humanized antibody sequence. The recombinant antibody was expressed according to step 7, and the affinity thereof to ET_{A}R was verified by FACS technique in step 9. Among the population of humanized antibodies that retain affinity to ET_{A}R, the antibodies with the best affinity were screened, and the variable region sequences thereof were further engineered by site-directed mutagenesis to further increase the affinity thereof to ET_{A}R.

### 6. Cloning and subcloning of ET_{A}R humanized antibody gene

The optimized humanized antibody heavy and light chain variable region sequences were synthesized by Genscript Biotechnology Co., Ltd, and during the synthesis, a restriction enzyme site Nhe1 was introduced into the 5' end of the heavy chain variable region and a restriction enzyme site Sal1 was introduced into the 3' end, so that the complete heavy chain variable region sequence was linked to the expression vector pTM5 into which the heavy chain constant region was incorporated; and similarly, a restriction enzyme site Nhe1 was introduced into the 5' end of the light chain variable region, and a restriction enzyme site Bsiw1 was introduced into the 3' end, so that the complete light chain variable region sequence was linked to the expression vector pTM5 into which the light chain constant region was incorporated.

### 7. Transient expression of antibody

5 × 10⁵/mL suspended HEK293 or CHO expressing cell strain was seeded into a spinner flask, and cultured under rotation for 24 h at 37°C and 5% CO₂ to reach a density of 1 × 10⁶/mL, and then used for transfection. During the transfection process, polyethylenimine (PEI) was used as a transfection medium, and mixed with DNA (the amount of DNA was 0.5 µg per 1 × 10⁶ cells, wherein the ratio of the light chain to the heavy chain of an antibody was 3 : 2), wherein the preferred mass mixing ratio of PEI to DNA was 3:1. The mixture of the two was added to the cell culture after 15 min of standing incubation. After receiving the mixture of PEI and DNA, the cells were cultured under rotation for 24 h at 37°C and 5% CO₂, and then 0.5% tryptone was added to the cell culture solution as an additive required for expression, and finally the cell supernatant was collected after the completion of the expression (over 96 h) for the purification and isolation of the antibody.

### 8. Purification and isolation of antibody

The collected cell supernatant was centrifuged at high speed (8000 rpm, 15 min) to remove cells and cell debris, and then filtered and clarified with a 0.45 µm filter membrane. The clarified supernatant was used for purification. The purification process was completed by a chromatograph. The supernatant first flowed through a Protein G affinity chromatography column, during which the antibody contained in the supernatant bound to the ligand of the Protein G affinity chromatography column and was retained in the column. Then an elution buffer with low pH value (less than or equal to 3.0) was used to wash the chromatography column to dissociate the antibody bound thereto, and the collected antibody eluate with low pH value was quickly neutralized with 1 M Tris-HCl to protect the antibody from denaturation and inactivation. The resulting antibody eluate was replaced with a PBS buffer system after 16 h of dialysis.

### 9. Analysis and verification of functional antibody by fluorescence-activated cell sorting (FACS)

10⁵ CHO-DHFR-hET_{A}R cells were digested and collected with PBS containing 10 mM EDTA, separately added into 1.5 mL EP tubes, the supernatant was discarded after centrifugation, and the negative control sample was resuspended in a flow loading buffer (PBS, 2% FBS). Positive treatment group cells in each tube were added with 200 µL of the antibody supernatant, and incubated at room temperature; after the completion of the incubation, the cells were centrifuged at 1500 rpm, the supernatant was discarded, the cell precipitate was washed once with a flow loading buffer, and centrifuged again, and the cells were resuspended; 1 : 50 diluted FITC labeled goat anti-mouse fluorescent secondary antibody (BD Pharmingen) was added to the cell resuspension solution at 200 µL/well, and the cells were incubated at room temperature for 30 min in the dark; and the cells were centrifuged, the supernatant was discarded, the cell was washed once with a flow loading buffer, and centrifuged, the cell precipitate was resuspended with a flow loading buffer for on-machine detection. The supernatant expressing recombinant anti-ET_{A}R functional antibody was specifically bound to CHO-DHFR-ET_{A}R cells expressing ET_{A}R. The gray peaks and dotted line peaks were negative controls; and the solid line peak corresponding to the hybridoma cell supernatant shifted obviously to the right.

### 10. Verification of functional antibody by calcium flux experiment

25000 CHO-DHFR- cells co-expressing hET_{A}R-Aequorin per well were seeded into a black 96-well cell culture plate, and cultured overnight at 37°C. On the next day, the cell supernatant was removed, the cells were added with 50 µL of a substrate coelenterazine h (Promega), incubated for 2 h in the dark, then added with 50 µL of the hybridoma cell supernatant or the purified antibody, and incubated for another 30 min. Endothelin-1 was perfused on a SpectraMax L microplate reader from Molecular Devices using an autosampler, the transient calcium flux change was detected within 40 s after perfusion, and the peak time and peak value were recorded. Different hybridoma supernatants showed different results of inhibiting intracellular human ET_{A}R mediated Ca²⁺ change, and the antibody A-1 significantly inhibited ET_{A}R mediated Ca²⁺ change. With the increase of the concentration of a recombinant anti-ET_{A}R functional antibody, the inhibitory effect of the antibody on intracellular human ET_{A}R mediated Ca²⁺ change was significantly enhanced.

### 11. Detection of biological activity of antibody A-1 in antagonizing different species of ET_{A}R in vitro by calcium flux experiment

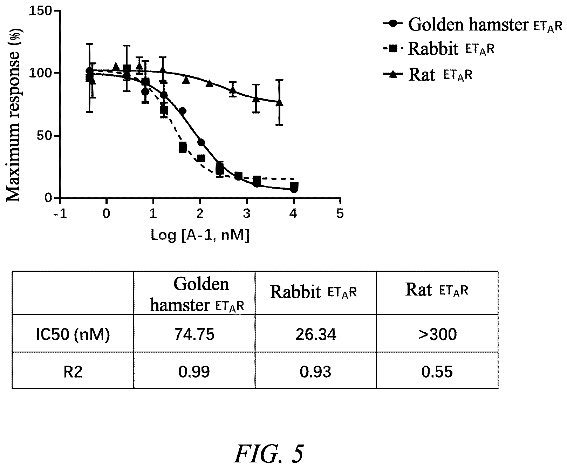

CHO-A1 cells co-expressing Aequorin, rabbit ET_{A}R, golden hamster ET_{A}R or rat ET_{A}R were digested with PBS containing 2 mM EDTA and collected. According to the counting results, each cell was inoculated into a sterile 96-well cell culture plate at 3.5 × 10⁴ cells/well or 4 × 10⁴ cells/well, respectively, at 100 µL/well, and the peripheral wells were supplemented with PBS at 100 µL/well. The cells were cultured overnight in a 37°C incubator. A tube of Coelenterazine-h was taken out at -20°C, and centrifuged after complete thawing to gather the liquid at the bottom of the EP tube. 3.2 mL of phenol red-free DMEM/F12 was added to a 5 mL centrifuge tube, and 100 µL of Colenterazine-h was added to the tube. After uniform mixing by inversion, 320 µL of the solution was dispensed into per well of a sample plate for later use. The cell culture plate was taken out, the original culture medium was sucked out, Coelenterazine-h was added at 50 µL/well with a multi-channel pipettor (performing in the dark), and the 96-well cell culture plate was placed in the dark for incubating at room temperature for 2-2.5 h. Antibody A-1 was diluted gradiently, 50 µL of a drug was added to 50 µL of coelenterazine per well in the dark, and two replicate wells were made for each concentration. Phenol red-free DMEM/F12 without antibody A-1 was used as blank control. After the completion of drug addition, the 96-well cell culture plate was incubated at room temperature for 30 min in the dark. At the end of incubation, the fluorescence intensity of each well was read by a microplate reader, wherein each well was filled with 100 µL of 10 nM ET-1. Plots were performed using prism software, which automatically calculated the IC50 values of the drugs and the curve fitting correlation coefficient R². As shown in FIG. 5, antibody A-1 can effectively antagonize ET-1-induced activation of rabbit ET_{A}R and golden hamster ET_{A}R, and cannot antagonize ET-1-induced activation of rat ET_{A}R.

### 12. Pharmacokinetic study of antibody A-1 in cynomolgus monkey

18 cynomolgus monkeys (9 females:9 males) were randomized into 3 groups, with 3 females and 3 males in each group. Each group of animals was given a single intravenous injection of 5 mg/kg, 15 mg/kg or 50 mg/kg antibody A-1, respectively, and bled before administration, immediately after administration (± 1 min), and 0.5 h, 2 h, 4 h, 8 h, 1 day, 2 days, 3 days, 4 days, 7 days, 14 days, 21 days and 28 days after administration. Serum was extracted by centrifugation, the concentration of the drug in the serum was detected by ELISA, and the half-life times of antibody A-1 in cynomolgus monkeys were determined by software analysis. As shown in Table 1, the half-life times of single intravenous injection of 5 mg/kg, 15 mg/kg or 50 mg/kg antibody A-1 in cynomolgus monkeys were 244.74 h, 173.15 h and 173.41 h, respectively.

**Table 1. Pharmacokinetics of single administration of antibody A-1 in cynomolgus monkey**

| Antibody A-1 | t_{1/2} | Cₘₐₓ |
|---|---|---|
| (mg/kg) | (h) | (µg/mL) |
| 5 | 244.74 ± 30.29 | 98.22 ± 16.81 |
| 15 | 173.15 ± 63.14 | 317.57 ± 38.19 |
| 50 | 173.41 ± 24.65 | 1,014.96 ± 73.63 |

### 13. Establishment of rabbit model with cisplatin-induced acute kidney injury to evaluate in vivo efficacy of antibody A-1

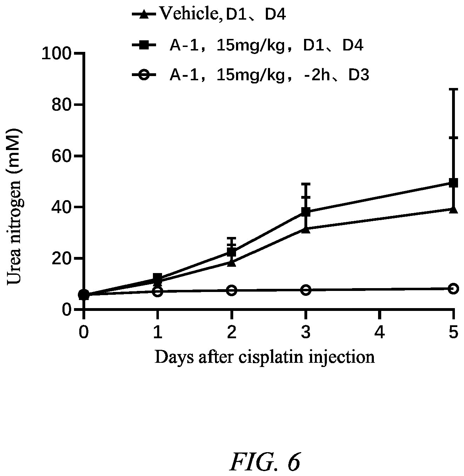

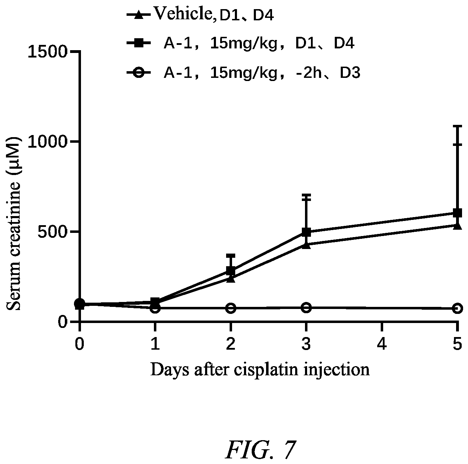

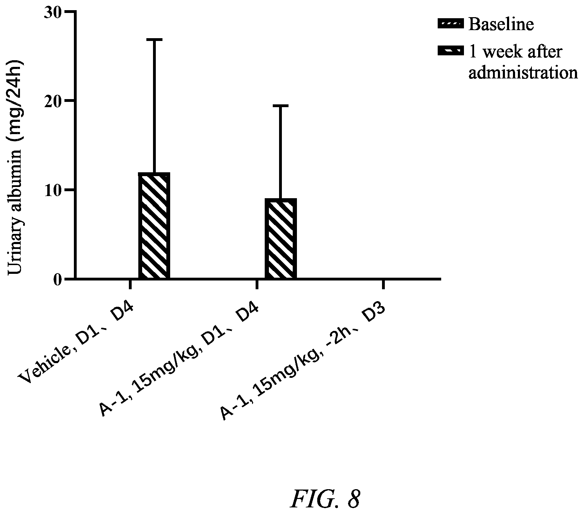

A rabbit model with acute kidney injury was induced by a single injection of cisplatin via ear vein at a dose of 5 mg/kg, and the efficacy of antibody A-1 injected intravenously twice a week was evaluated in this model. 24 animals were randomly selected. Before cisplatin injection or before administration of antibody A-1, rabbits were weighed, 24-h urine and serum of the rabbits were collected to detect the contents of urinary total protein (U-TP), urinary albumin (U-Alb), urea nitrogen (BUN) and serum creatinine (sCr). The 24 animals were randomly grouped into 3 groups according to their body weights, with 8 animals in each group. The first administration time of the animals in group 2 was 24 h (D1) after cisplatin injection, and the second administration time was 4 days (D4) after cisplatin injection. The first administration time of the animals in group 3 was 2 h before cisplatin injection (-2 h), and the second administration time was 3 days (D3) after cisplatin injection. 1 day, 2 days, 3 days and 5 days after cisplatin injection, serum was collected again to detect BUN and sCr. 24-h urine was collected at the end of the experiment to detect U-TP and U-Alb. As shown in FIG. 6, FIG. 7, Table 2 and Table 3, treatment with antibody A-1 at -2 h and D3 significantly reduced or blocked cisplatin-induced increases in BUN and sCr. As shown in FIG. 8 and Table 4, treatment with antibody A-1 at -2 h and D3 reduced or blocked cisplatin-induced increases in U-TP and U-Alb. The above data showed that antibody A-1 (-2 h and D3) had superior renal protection effect in the rabbit model with cisplatin-induced acute kidney injury.

**Table 2. BUN (Mean ± SD) of rabbits in each group at different time points**

| Group | D0 | D1 | D2 | D3 | D5 |
|---|---|---|---|---|---|
| Group 1, vehicle IV, D1, 4 | 5.76 ± 0.86 | 10.94 ± 2.64 | 18.54 ± 6.68 | 31.53 ± 12.27 | 39.27 ± 27.82 |
| Group 2, antibody A-1 15 mg/kg, IV, D1, 4 | 5.57 ± 0.64 | 11.97 ± 1.77## | 22.46 ± 5.40## | 38.16 ± 10.87## | 49.56 ± 36.44# |
| Group 3, antibody A-1 15 mg/kg, -2 h, D3 | 5.80 ± 0.91 | 7.07 ± 0.94** | 7.50 ± 0.86** | 7.64 ± 0.83** | 8.13 ± 1.21* |

| | | | | | |
|---|---|---|---|---|---|
| Remarks: comparing group 2 with group 1, from D1 to D5, P > 0.05; comparing group 3 with group 1, * indicates P < 0.05, ** indicates P < 0.01; comparing group 2 with group 3, # indicates P < 0.05, ## indicates P < 0.01. | | | | | |

**Table 3. sCr (Mean ± SD) of rabbits in each group at different time points**

| Group | D0 | D1 | D2 | D3 | D5 |
|---|---|---|---|---|---|
| Group 1, vehicle IV, D1, 4 | 99.14 ± 8.09 | 104.24 ± 15.76 | 242.65 ± 128.94 | 429.04 ± 248.30 | 537.39 ± 445.35 |
| Group 2, antibody A-1 15 mg/kg, IV, D1, 4 | 93.10 ± 9.56 | 111.50 ± 12.13## | 283.55 ± 78.84## | 498.80 ± 204.91## | 605.28 ± 481.84# |
| Group 3, antibody A-1 15 mg/kg, -2 h, D3 | 100.88 ± 9.37 | 76.71 ± 10.03** | 75.65 ± 9.53* | 78.66 ± 10.99* | 74.69 ± 11.85 |

| | | | | | |
|---|---|---|---|---|---|
| Remarks: comparing group 2 with group 1, from D1 to D5, P > 0.05; comparing group 3 with group 1, * indicates P < 0.05, ** indicates P < 0.01; comparing group 2 with group 3, # indicates P < 0.05, ## indicates P < 0.01. | | | | | |

**Table 4. U-TP and U-Alb (Mean ± SD) of rabbits in each group at different time points**

| Group | U-TP (mg/24 h) | | U-Alb (mg/24 h) | |
|---|---|---|---|---|
| | Baseline | 1 week after administration | Baseline | 1 week after administration |
| Group 1 Vehicle, IV, D1, 4 | 12.78 ± 2.26 | 94.42 ± 81.96 | 0.00 ± 0.00 | 11.95 ± 14.89 |
| Group 2 Antibody A-1, 15 mg/kg, IV, D1, 4 | 11.18 ± 3.12 | 62.68 ± 50.74 | 0.00 ± 0.00 | 9.02 ± 10.44 |
| Group 3 Antibody A-1, 15 mg/kg, -2 h, D3 | 12.25 ± 3.41 | 20.06 ± 3.39* | 0.00 ± 0.00 | 0.00 ± 0.00 |

| | | | | |
|---|---|---|---|---|
| Remarks: comparing group 2 with group 1, from D1 to D5, P > 0.05; comparing group 3 with group 1, * indicates P < 0.05, ** indicates P < 0.01; comparing group 2 with group 3, # indicates P < 0.05, ## indicates P < 0.01. | | | | |

**Table 5. U-TP, U-Alb and urine volume (Mean ± SD) of golden hamsters in each group at different time points**

| Group | U-TP (mg/24 h) | | U-Alb (mg/24 h) | | Urine volume (mL/24 h) | |
|---|---|---|---|---|---|---|
| | Baseline | 2 weeks after administration | Baseline | 2 weeks after administration | Baseline | 2 weeks after administration |
| Group 1, blank group | 7.6 ± 3.1 | 5.9 ± 2.4 | 0.06 ± 0.04 | 0.18 ± 0.13 | 5.8 ± 4.5 | 6 ± 4.6 |
| Group 2, model group | 9.4 ± 9 | 21.1 ± 5.3^{###} | 0.12 ± 0.1 | 1.93 ± 0.77^{###} | 7 ± 2.7 | 29.1 ± 9.1^{###} |
| Group 3, antibody A-1, 6 mg/kg, i.p., BIW*2 | 8.2 ± 7.6 | 17.8 ± 7.6^{##} | 0.07 ± 0.04 | 1.35 ± 1.14 | 5.8 ± 2.7 | 20.2 ± 9.8^{##}* |
| Group 4, antibody A-1, 18 mg/kg, i.p., BIW*2 | 11.3 ± 6.9 | 13 6* | 0.07 ± 0.09 | 1.34 ± 0.84^{#} | 8.7 ± 2.9 | 16.8 ± 15.1^{#}** |
| Group 5, antibody A-1, 54 mg/kg, i.p., BIW*2 | 6.4 ± 4.9 | 13.5 ± 4.6^{#}* | 0.03 ± 0.02 | 1.37 ± 0.58^{##} | 5.3 ± 3.2 | 11.5 ± 6.1*** |

| | | | | | | |
|---|---|---|---|---|---|---|
| Remarks: compared to group 1, # indicates P < 0.05, ## indicates P < 0.01, ### indicates P < 0.001; compared to group 2, * indicates P < 0.05, ** indicates P < 0.01, *** indicates P < 0.001. | | | | | | |

### 14. Establishment of golden hamster model with diabetic nephropathy to evaluate in vivo efficacy of antibody A-1

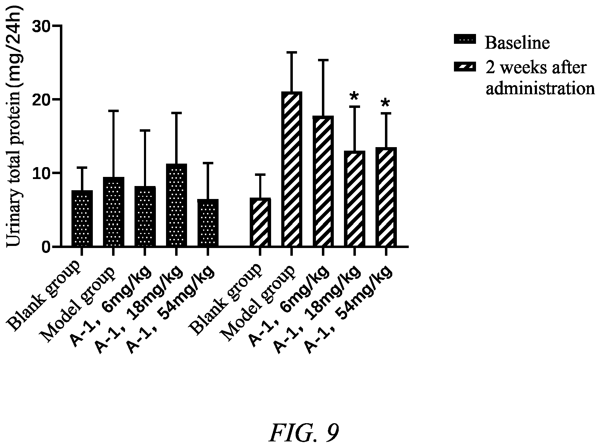

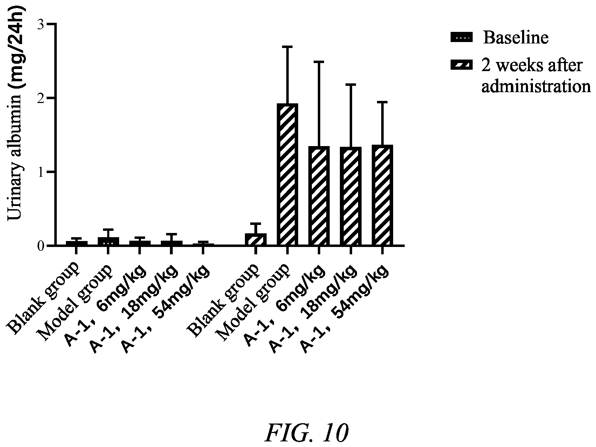

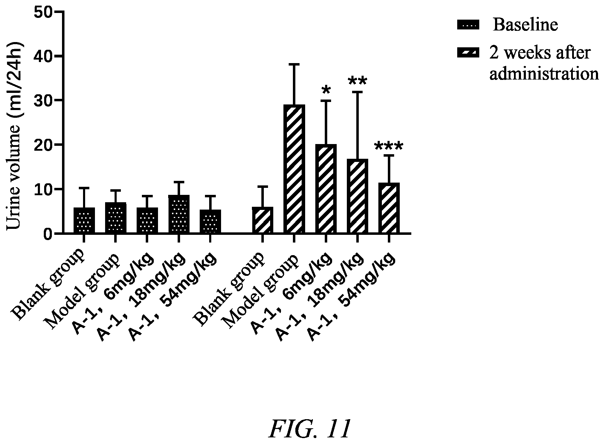

A golden hamster model with diabetic nephropathy was induced by intraperitoneal injection of streptozotocin (STZ) at a dose of 30 mg/kg for 3 consecutive days in combination with a high-fat high-cholesterol diet, and the efficacy of antibody A-1 injected intraperitoneally twice a week was evaluated in this model. 50 golden hamsters were randomly selected. Urine was collected to detect urinary creatinine (U-Cr), U-TP and U-Alb, and serum was collected to detect fasting blood glucose, BUN and sCr. The animals were randomly grouped according to their body weights, with 10 animals in the blank group. The remaining animals were intraperitoneally injected with STZ at a dose of 30 mg/kg for 3 consecutive days, and then grouped on day 4, with 10 animals in each group. After grouping, vehicle (blank group and model group), and 6 mg/kg, 18 mg/kg or 54 mg/kg antibody A-1 were injected separately, and the day of administration was defined as D0. The animals were fed with common diet for 10 days. On day 11, the diet was changed into a high-fat high-cholesterol diet until the end of the experiment. Two weeks after administration, urine was collected to detect U-Cr, U-TP and U-Alb. As shown in FIG. 9 to FIG. 11 and Table 5, the 24-h urine volume, total urine protein and urinary albumin increased in the model group after STZ injection compared to the blank group, suggesting successful modeling of the early diabetic nephropathy model; and compared with the model group, antibody A-1 significantly reduced the 24-h urine volume and total urine protein in diabetic nephropathy animals, and at the same time, the 24-h urine albumin in diabetic nephropathy animals after the treatment with antibody A-1 also decreased by approximately 30%, showing a good renal protection effect.

The above examples are provided to provide one of ordinary skill in the art with sufficient disclosure and illustration of how to make and use the claimed embodiments, and are not meant to limit the scope of the disclosure herein. For one skilled in the art, obvious modifications all fall within the scope of the claims herein. All publications, patents and patent applications cited in this description are incorporated herein by reference as if each publication, patent or patent application is specifically and individually incorporated herein by reference.

## Claims

1. An antibody capable of specifically binding to ET_{A}R, **characterized in that** the antibody comprises one, two, three, four, five or six amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30;
b. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48;
c. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68;
d. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90;
e. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114; and
f. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

2. The antibody of claim 1 capable of specifically binding to ET_{A}R, **characterized in that** the antibody comprises one or two amino acid sequences, wherein each amino acid sequence is independently selected from the amino acid sequences listed below:
a. light chain CDR1 amino acid sequences: SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, and SEQ ID NO: 30; and
b. heavy chain CDR1 amino acid sequences: SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, and SEQ ID NO: 90.

3. The antibody of claim 1 capable of specifically binding to ET_{A}R, **characterized in that** the antibody comprises one or two amino acid sequences, wherein each amino acid sequence is independently selected from the amino acid sequences listed below:
a. light chain CDR2 amino acid sequences: SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, and SEQ ID NO: 48; and
b. heavy chain CDR2 amino acid sequences: SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, and SEQ ID NO: 114.

4. The antibody of claim 1 capable of specifically binding to ET_{A}R, **characterized in that** the antibody comprises one or two amino acid sequences, wherein each amino acid sequence is independently selected from the amino acid sequences listed below:
a. light chain CDR3 amino acid sequences: SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, and SEQ ID NO: 68; and
b. heavy chain CDR3 amino acid sequences: SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, and SEQ ID NO: 136.

5. The antibody of claim 1 capable of specifically binding to ET_{A}R, **characterized in that** the antibody comprises one or two amino acid sequences, wherein each amino acid sequence is independently selected from the amino acid sequences listed below: SEQ ID NO: 8, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 44, SEQ ID NO: 46, SEQ ID NO: 48, SEQ ID NO: 50, SEQ ID NO: 62, SEQ ID NO: 64, SEQ ID NO: 66, and SEQ ID NO: 68.

6. The antibody of claim 1 capable of specifically binding to ET_{A}R, **characterized in that** the antibody comprises one or two amino acid sequences, wherein each amino acid sequence is independently selected from the amino acid sequences listed below: SEQ ID NO: 70, SEQ ID NO: 82, SEQ ID NO: 84, SEQ ID NO: 86, SEQ ID NO: 88, SEQ ID NO: 90, SEQ ID NO: 92, SEQ ID NO: 104, SEQ ID NO: 106, SEQ ID NO:108, SEQ ID NO: 110, SEQ ID NO: 112, SEQ ID NO: 114, SEQ ID NO: 116, SEQ ID NO: 128, SEQ ID NO: 130, SEQ ID NO: 132, SEQ ID NO: 134, and SEQ ID NO: 136.

7. The antibody of claim 1 capable of specifically binding to ET_{A}R, **characterized in that** the antibody comprises a combination of light chain and heavy chain CDR3 amino acid sequences independently selected from the list below: SEQ ID NO: 50 and SEQ ID NO: 116, SEQ ID NO: 62 and SEQ ID NO: 128, SEQ ID NO: 62 and SEQ ID NO: 130, SEQ ID NO: 64 and SEQ ID NO: 132, SEQ ID NO: 66 and SEQ ID NO: 134, and SEQ ID NO: 68 and SEQ ID NO: 136.

8. The antibody of claim 1 capable of specifically binding to ET_{A}R, **characterized in that** the antibody comprises one or two amino acid sequences, wherein each amino acid sequence is independently selected from the amino acid sequences listed below:
a. light chain variable domain amino acid sequences: SEQ ID NO: 138, SEQ ID NO: 140, SEQ ID NO: 142, SEQ ID NO: 144, SEQ ID NO: 146, SEQ ID NO: 148, SEQ ID NO: 150, SEQ ID NO: 152, SEQ ID NO: 154, SEQ ID NO: 156, SEQ ID NO: 158, SEQ ID NO: 160, SEQ ID NO: 162, and SEQ ID NO: 164; and
b. heavy chain variable domain amino acid sequences: SEQ ID NO: 166, SEQ ID NO: 168, SEQ ID NO: 170, SEQ ID NO: 172, SEQ ID NO: 174, SEQ ID NO: 176, SEQ ID NO: 178, SEQ ID NO: 180, SEQ ID NO: 182, SEQ ID NO: 184, SEQ ID NO: 186, SEQ ID NO: 188, SEQ ID NO: 190, and SEQ ID NO: 192.

9. The antibody of claim 1 capable of specifically binding to ET_{A}R, **characterized in that** the polynucleotide coding sequence of the antibody comprises one or two polynucleotide sequences, wherein each polynucleotide sequence is independently selected from the polynucleotide sequences listed below:
a. light chain variable domain polynucleotide coding sequences: SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, and SEQ ID NO: 163; and
b. heavy chain variable domain polynucleotide coding sequences: SEQ ID NO: 165, SEQ ID NO: 167, SEQ ID NO: 169, SEQ ID NO: 171, SEQ ID NO: 173, SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 189, and SEQ ID NO: 191.

10. The antibody of claim 1 capable of specifically binding to ET_{A}R, **characterized in that** the antibody comprises a combination of amino acid sequences independently selected from the list below: SEQ ID NO: 138 and SEQ ID NO: 166, SEQ ID NO: 140 and SEQ ID NO: 168, SEQ ID NO: 142 and SEQ ID NO: 170, SEQ ID NO: 144 and SEQ ID NO: 172, SEQ ID NO: 146 and SEQ ID NO: 174, SEQ ID NO: 148 and SEQ ID NO: 176, SEQ ID NO: 150 and SEQ ID NO: 178, SEQ ID NO: 152 and SEQ ID NO: 180, SEQ ID NO: 154 and SEQ ID NO: 182, SEQ ID NO: 156 And SEQ ID NO: 184, SEQ ID NO: 158 and SEQ ID NO: 186, SEQ ID NO: 160 and SEQ ID NO: 188, SEQ ID NO: 162 and SEQ ID NO: 190, and SEQ ID NO: 164 and SEQ ID NO: 192.

11. The antibody of claim 1 capable of specifically binding to ET_{A}R, **characterized in that** the antibody comprises an amino acid sequence independently selected from the list below: SEQ ID NO: 138, SEQ ID NO: 150, SEQ ID NO: 152, SEQ ID NO: 154, SEQ ID NO: 156, SEQ ID NO: 158, SEQ ID NO: 160, SEQ ID NO: 162, and SEQ ID NO: 164.

12. The antibody of claim 1 capable of specifically binding to ET_{A}R, **characterized in that** the antibody comprises an amino acid sequence independently selected from the list below: SEQ ID NO: 166, SEQ ID NO: 178, SEQ ID NO: 180, SEQ ID NO: 182, SEQ ID NO: 184, SEQ ID NO: 186, SEQ ID NO: 188, SEQ ID NO: 190, and SEQ ID NO: 192.

13. The antibody of claim 1 capable of specifically binding to ET_{A}R, **characterized in that** the antibody comprises a combination of light chain and heavy chain variable region amino acid sequences independently selected from the list below: SEQ ID NO: 138 and SEQ ID NO: 166, SEQ ID NO: 150 and SEQ ID NO: 178, SEQ ID NO: 152 and SEQ ID NO: 180, SEQ ID NO: 154 and SEQ ID NO: 182, SEQ ID NO: 156 and SEQ ID NO: 184, SEQ ID NO: 158 and SEQ ID NO: 186, SEQ ID NO: 160 and SEQ ID NO: 188, SEQ ID NO: 162 and SEQ ID NO: 190, and SEQ ID NO: 164 and SEQ ID NO: 192.

14. The antibody of claim 1 capable of specifically binding to ET_{A}R, **characterized in that** the antibody comprises an amino acid sequence SEQ ID NO: 138 or SEQ ID NO: 166.

15. The antibody of claim 1 capable of specifically binding to ET_{A}R, **characterized in that** the antibody comprises a combination of amino acid sequences SEQ ID NO: 138 and SEQ ID NO: 166.

16. The antibody of claim 1 capable of specifically binding to ET_{A}R, **characterized in that** the antibody comprises one or two amino acid sequences, wherein each amino acid sequence is independently selected from the amino acid sequences listed below:
a. light chain constant region amino acid sequences: SEQ ID NO: 194 and SEQ ID NO: 196; and
b. a heavy chain constant region amino acid sequence: SEQ ID NO: 198.

17. The antibody of claim 1 capable of specifically binding to ET_{A}R, **characterized in that** the antibody comprises a murine antibody or a humanized antibody.

18. The antibody of claim 1 capable of specifically binding to ET_{A}R, **characterized in that** the antibody comprises a monoclonal antibody.

19. The antibody of claim 1 capable of specifically binding to ET_{A}R, **characterized in that** the antibody comprises a monoclonal antibody, and the monoclonal antibody comprises SEQ ID NO: 138 and SEQ ID NO: 166, SEQ ID NO: 150 and SEQ ID NO: 178, SEQ ID NO: 152 and SEQ ID NO: 180, SEQ ID NO: 154 and SEQ ID NO: 182, SEQ ID NO: 156 and SEQ ID NO: 184, SEQ ID NO: 158 and SEQ ID NO: 186, SEQ ID NO: 160 and SEQ ID NO: 188, SEQ ID NO: 162 and SEQ ID NO: 190, and SEQ ID NO: 164 and SEQ ID NO: 192.

20. The antibody of claim 1 capable of specifically binding to ET_{A}R, **characterized in that** the antibody has an IC₅₀ value of approximately 1 nM to 200 nM or 10 nM to 100 nM in reducing human endothelin signaling.

21. A pharmaceutical composition, **characterized by** comprising the antibody of claim 1 capable of specifically binding to ET_{A}R which is mixed with a pharmaceutically acceptable carrier.

22. A polynucleotide, **characterized by** encoding the antibody of claim 1 capable of specifically binding to ET_{A}R.

23. A recombinant expression vector, **characterized by** comprising the nucleic acid of claim 22.

24. A host cell, **characterized by** comprising the vector of claim 23.

25. A method for producing an antibody capable of specifically binding to ET_{A}R, **characterized in that** the method comprises culturing the host cell of claim 24 under conditions that allow the expression of the antibody.

26. A method for treating, preventing or ameliorating one or more symptoms of diabetic nephropathy in a subject, **characterized by** comprising administering to the subject a therapeutically effective amount of the pharmaceutical composition of claim 21.

27. A method for treating, preventing or ameliorating one or more symptoms of chronic nephropathy in a subject, **characterized by** comprising administering to the subject a therapeutically effective amount of the pharmaceutical composition of claim 21.

28. A method for treating, preventing or ameliorating one or more symptoms of a disease accompanied by proteinuria in a subject, **characterized by** comprising administering to the subject a therapeutically effective amount of the pharmaceutical composition of claim 21.

29. A method for treating, preventing or ameliorating one or more symptoms of a disease accompanied by a decrease in glomerular filtration rate in a subject, **characterized by** comprising administering to the subject a therapeutically effective amount of the pharmaceutical composition of claim 21.

30. The method of any one of claims 26 to 29, **characterized in that** the subject is a human.
